# FASCICULE DE BREVET EUROPEEN

(11) **EP 0 514 264 B1**
(45) Date de publication et mention de la délivrance du brevet: **15.11.1995**
(21) Numéro de dépôt: 92401306.3
(22) Date de dépôt: 13.05.1992
(51) Int. Cl.: C07C 65/26, C07C 65/40, C07C 65/17, C07C 65/19

(54) **Composés bi-aromatiques dérivés d'un motif salicylique, leur procédé de préparation et leur utilisation en médecine humaine et vétérinaire ainsi qu'en cosmétique**
Bi-aromatische, von Salizylkonfiguration enthaltende Verbindungen abgeleitete Verbindungen, Verfahren zu ihrer Herstellung und ihre Verwendung in menschlicher und tierischer Medizin und in der Kosmetik
Biaromatic compounds derived from compounds having a salicylic configuration, their preparation process and their use in human and veterinary medicine and in cosmetics

(30) Priorité: 13.05.1991 FR 9105747
(43) Date de publication de la demande: 19.11.1992
(73) Titulaire: CENTRE INTERNATIONAL DE RECHERCHES DERMATOLOGIQUES GALDERMA, ( CIRD GALDERMA), 06560 Valbonne (FR)
(72) Inventeur: Bernardon, Jean Michel, 06650 Nice (FR)
(74) Mandataire: Stalla-Bourdillon, Bernard

(56) Documents cités:
- EP-A- 0 154 928
- FR-A- 2 172 868
- GB-A- 1 566 497

## Description

La présente invention a pour objet de nouveaux composés bi-aromatiques dérivés d'un motif salicylique, leur procédé de préparation et leur utilisation en médecine humaine et vétérinaire et en cosmétique.

Ces nouveaux composés trouvent une application dans le traitement topique et systémique des affections dermatologiques liées à un désordre de la kératinisation (différenciation-prolifération) et d'affections dermatologiques, ou autres, à composantes inflammatoires et/ou immunoallergiques et dans les maladies de dégénérescence du tissu conjonctif, et présentent une activité anti-tumorale. En outre, ces composés peuvent être utilisés dans le traitement de l'atopie, qu'elle soit cutanée ou respiratoire et du psoriasis rhumatoïde.

Ils trouvent également une application dans le domaine ophtalmologique, notamment dans le traitement des cornéopathies.

Les composés selon l'invention peuvent être représentés par la formule générale suivante : 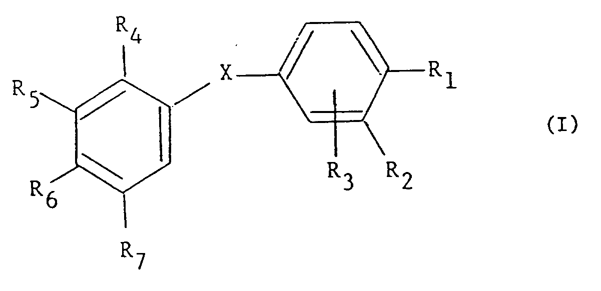
dans laquelle :
R₁ représente le radical - CH₃, le radical - CH₂OH, le radical - COR₈, ou le radical - CH₂OCOR₉,
R₈ représentant un atome d'hydrogène, OH, - OR₁₀, 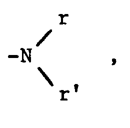
ou un radical alkyle inférieur,
R₁₀ représentant un radical alkyle ayant de 1 à 20 atomes de carbone, un radical alkényle ayant de 2 à 20 atomes de carbone, un radical aryle ou aralkyle,
r et r', identiques ou différents, représentant un atome d'hydrogène, un radical alkyle inférieur, un radical aryle, un radical aralkyle, un reste d'α-aminoacide, un reste de sucre ou un hétérocycle ou r et r' pris ensemble formant un hétérocycle,
R₉ représentant un radical alkyle ayant de 1 à 20 atomes de carbone, un radical alkényle ayant de 2 à 20 atomes de carbone ou un reste de sucre,
R₂ et R₃ représentent - OR₁₁ ou - OCOR₁₁
R₁₁ représentant un atome d'hydrogène, un radical alkyle inférieur, un radical fluoroalkyle ayant de 1 à 6 atomes de carbone et de 3 à 7 atomes de fluor, un radical aryle ou un radical aralkyle,
R₃ pouvant représenter en outre un atome d'hydrogène,
R₄ représente un atome d'hydrogène, OH, un radical alkyle inférieur, un radical alkoxy ayant de 1 à 6 atomes de carbone, un atome de fluor, de chlore ou le groupe - CF₃,
R₅ et R₇ représentent un atome d'hydrogène, OH, un radical alkoxy ayant de 1 à 6 atomes de carbone, un radical alkyle α-substitué ayant de 3 à 12 atomes de carbone ou un radical alkyle α-α'-disubstitué ayant de 4 à 12 atomes de carbone, un radical cycloalkyle ayant de 3 à 12 atomes de carbone, un radical mono ou polycyclique ayant de 5 à 12 atomes de carbone lié au noyau phényle par un carbone tertiaire, R₅ et R₇ ne pouvant représenter simultanément OH ou alcoxy,
R₆ représente un atome d'hydrogène, OH, un radical alkyle inférieur, un radical alkoxy ayant de 1 à 6 atomes de carbone, un radical cycloalkyle ayant de 3 à 12 atomes de carbone, un radical monohydroxyalkyle, un radical polyhydroxyalkyle, un atome de fluor, un atome de chlore, un radical alkényle ayant de 2 à 6 atomes de carbone ou un radical alkényloxy ayant de 2 à 6 atomes de carbone, R₄, R₅, R₆ et R₇ ne pouvant représenter simultanément un atome d'hydrogène,
R₅ et R₆ ou R₆ et R₇ pris ensemble peuvent former avec le cycle aromatique adjacent un cycle à 5 ou 6 chainons éventuellement substitué par des groupes méthyle et/ou éventuellement interrompu par un atome d'oxygène ou de soufre,
X est un radical divalent qui peut être lu de gauche à droite ou inversement choisi dans le groupe constitué par :
(i) -C(R₁₃R₁₄)-C(R₁₆R₁₈)-W-
(ii) -C(R₁₄R₁₆)-W-C(R₁₈R₁₉)-
(iii) -C(R₁₃R₁₄)-C(R₁₅R₁₆)-C(R₁₈R₂₀)-
(iv) -CR₁₇=CR₂₁-C(R₁₃R₁₄)-
dans lesquels :
W représente un atome d'oxygène, le groupe -NR₁₂ ou le groupe S(O)n, n étant 0, 1 ou 2,
R₁₃, R₁₅ et R₂₀ représentent un atome d'hydrogène, le radical - OR₁₁, - OCOR₁₁, - NHCOR₁₁, un radical 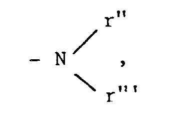
un radical aralkyle, un radical alkyle inférieur, un radical monohydroxyalkyle ou un radical polyhydroxyalkyle,
r'' et r''' identiques ou différents, représentant un atome d'hydrogène, un radical alkyle inférieur, un radical alkényle ayant de 2 à 6 atomes de carbone ou un radical alkynyle ayant de 2 à 6 atomes de carbone,
R₁₄, R₁₆, R₁₈ et R₁₉ représentent un atome d'hydrogène, un radical aralkyle, un radical alkyle inférieur, un radical monohydroxyalkyle ou polyhydroxyalkyle,
lorsque X représente (i), R₁₃ et R₁₄ peuvent former un groupe = N - OR₁₁ ou un groupe = N - OCOR₁₁,
lorsque X représente (iii) ou (iv), R₁₄, R₁₆ et R₁₈ peuvent également représenter le radical - OR₁₁ ou le radical - OCOR₁₁, ou encore R₁₃, R₁₄ ou R₁₅, R₁₆ pris ensemble peuvent former un groupe = NOR₁₁ ou un groupe = N - OCOR₁₁,
R₁₂ représentant un atome d'hydrogène, un radical alkyle inférieur, un radical aralkyle, un radical alkényle ayant de 2 à 6 atomes de carbone, un radical alkynyle ayant de 2 à 6 atomes de carbone ou un radical fluoroalkyle ayant de 1 à 6 atomes de carbone et de 3 à 7 atomes de fluor,
R₁₇ représentant un atome d'hydrogène, un groupe hydroxyle, un radical alkyle inférieur ou un radical alkoxy ayant de 1 à 6 atomes de carbone,
R₂₁ représentant un atome d'hydrogène ou un radical alkyle inférieur,
et les sels des composés de formule (I) lorsque R₁ représente une fonction acide carboxylique ou lorsque R₁₃, R₁₅ ou R₂₀ représente une fonction amine et les isomères optiques des composés de formule (I).

Lorsque les composés selon l'invention se présentent sous forme de sels, dans le cas où R₁ représente une fonction carboxylique, il s'agit de sels d'un métal alcalin ou alcalino-terreux ou encore de zinc ou d'une amine organique, dans le cas où R₁₃ ou R₁₅ ou R₂₀ représente un groupe amine il s'agit de sels pharmaceutiquement ou cosmétiquement acceptables formés par addition d'un acide minéral ou organique choisi parmi l'acide chlorhydrique, sulfurique, acétique, citrique, fumarique, hémisuccinique, maléique ou mandélique,

Par radical alkyle inférieur, on entend un radical ayant de 1 à 6 atomes de carbone et de préférence les radicaux méthyle, éthyle, isopropyle, butyle et tertiobutyle.

Par radical alkoxy ayant de 1 à 6 atomes de carbone, on doit notamment entendre un radical méthoxy, éthoxy, isopropoxy ou butoxy.

Par radical alkyle α-substitué ayant de 3 à 12 atomes de carbone, on doit notamment entendre un radical isopropyle, 1-méthylpropyle ou 1-éthylpropyle.

Par radical alkyle α,α'-disubstitué ayant de 4 à 12 atomes de carbone, on doit notamment entendre un radical tert-butyle, 1,1-diméthyl propyle, 1-méthyl 1-éthyl propyle, 1-méthyl 1-éthyl hexyle ou 1,1-diméthyl décyle.

Par radical monohydroxyalkyle, on doit entendre un radical ayant de 1 à 6 atomes de carbone, notamment un radical 2-hydroxyéthyle, 2-hydroxypropyle ou 3-hydroxypropyle.

Par radical polyhydroxyalkyle, on doit entendre un radical contenant de 2 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on doit entendre un radical phényle éventuellement substitué par au moins un atome d'halogène, un hydroxyle ou une fonction nitro.

Par radical aralkyle, on doit entendre le radical benzyle ou phénéthyle éventuellement substitué par au moins un atome d'halogène, un hydroxyle ou une fonction nitro.

Par radical cycloalkyle ayant de 3 à 12 atomes de carbone, on doit entendre notamment un radical cyclopentyle ou cyclohexyle.

Par radical cycloalkyle mono ou polycyclique ayant de 5 à 12 atomes de carbone dont le carbone de liaison est trisubstitué, on doit entendre le radical 1-méthyl cyclohexyle ou 1-adamantyle.

Par radical alkényloxy ayant de 2 à 6 atomes de carbone on doit entendre des radicaux linéaires ou ramifiés notamment allyloxy et vinyloxy.

Par radical alkényle ayant de 2 à 6 atomes de carbone, on doit entendre notamment les radicaux vinyle, allyle ou 2-butènyle.

Par radical alkynyle ayant de 2 à 6 atomes de carbone, on doit entendre notamment le radical propargyle.

Par radical fluoroalkyle ayant de 1 à 6 atomes de carbone et de 3 à 7 atomes de fluor on entend en particulier les groupes CF₃ et C₂F₅.

Lorsque R₉ ou R₁₀ représente un radical alkyle ayant de 1 à 20 atomes de carbone ou un radical alkényle ayant de 2 à 20 atomes de carbone on doit entendre des radicaux linéaires ou ramifiés éventuellement substitués par un ou plusieurs groupes hydroxyles ou un ou plusieurs atomes de fluor.

Par reste d'aminoacide on doit entendre un reste dérivant par exemple de l'un des 20 aminoacides de configuration L ou D (ou leur mélange racémique) constitutifs de protéines de mammifères.

Par reste d'un sucre on doit entendre un reste dérivant par exemple du glucose, galactose ou mannose.

Par hétérocycle, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Parmi les composés de formule (I) ci-dessus, on peut notamment citer les suivants :
1) Acide 2-hydroxy-4-[2-hydroxy-2-(5,6,7,8-tétrahydro-5,5,8,8-tétraméthyl-2-naphtyl)éthoxy]benzoïque ;
2) 2-hydroxy-4-[2-hydroxy-2-(5,6,7,8-tétrahydro -5,5,8,8,-tétraméthyl-2-naphtyl)éthoxy]benzoate de méthyle ;
3) Acide 2-hydroxy-4-[2-hydroxyimino-2-(5,6,7,8-tétrahydro-5,5,8,8-tétraméthyl-2-naphtyl)éthoxy]benzoïque ;
4) Acide 2-acétyloxy-4-[2-acétyloxy-2-(5,6,7,8-tétrahydro-5,5,8,8-tétraméthyl-2-naphtyl)éthoxy]benzoïque ;
5) Acide 2-hydroxy-4-[2-acétyloxy-2-(5,6,7,8-tétrahydro-5,5,8,8-tétraméthyl-2-naphtyl)éthoxy]benzoïque ;
6) Acide 2-acétyloxy-4-[2-hydroxy-2-(5,6,7,8-tétrahydro-5,5,8,8-tétraméthyl-2-napthyl)éthoxy]benzoïque ;
7) Acide-2-hydroxy-4- [2-(5,6,7,8-tétrahydro-5,5,8,8-tétraméthyl -2-naphtyl)éthoxy]benzoïque ;
8) 2-hydroxy-4-[2-hydroxy-2-(5,6,7,8-tétrahydro-5,5,8,8-tétraméthyl-2-naphtyl)éthoxy]benzyl alcool ;
9) Acétate de 2-acétyloxy-4-[2-hydroxy-2-(5,6,7,8-tétrahydro-5,5,8,8-tétraméthyl-2-naphtyl)éthoxy]benzyl alcool ;
10) Piperidinylamide de l' acide 2-hydroxy-4-[2-hydroxy-2-(5,6,7,8-tétrahydro-5,5,8,8-tétraméthyl-2-naphtyl)éthoxy]benzoïque ;
11) Morpholinylamide de l'acide 2-hydroxy-4-[2-hydroxy-2-(5,6,7,8-tétrahydro-5,5,8,8-tétraméthyl-2-naphtyl)éthoxy]benzoïque.
12) 2-hydroxy-4-[2-hydroxy-2-(5,6,7,8-tétrahydro-5,5,8,8-tétraméthyl-2-naphtyl)éthoxy]benzamide ;
13) N-éthyl amide de l'acide 2-hydroxy-4-[2-hydroxy-2-(5,6,7,8-tétrahydro-5,5,8,8-tétraméthyl-2-naphtyl)éthoxy]benzoïque ;
14) 2-hydroxy-4-[2-hydroxy-2-(4,4-diméthylthiochroman-6-yl) éthoxy]benzoate de méthyle ;
15) Acide 2-hydroxy-4-[2-hydroxy-2-(4,4-diméthylthiochroman-6-yl) éthoxy]benzoïque ;
16) Acide 2-hydroxy-4-[3-hydroxy-3-(5,6,7,8-tétrahydro-5,5,8,8-tétraméthyl-2-naphtyl)propyl]benzoïque ;
17) Acide 2-hydroxy-4-[2-hydroxy-2-(3,5-di-tert-butyl-4-hydroxyphényl)éthoxy]benzoïque ;
18) 2-hydroxy-4-[2-hydroxy-2-(5,6,7,8-tétrahydro-5,5,8,8-tétraméthyl-2-naphtyl)éthoxy]toluène ;
19) 2,6-dihydroxy-4-[2-hydroxy-2-(5,6,7,8-tétrahydro-5,5,8,8-tétraméthyl-2-naphtyl)éthoxy]benzoate de méthyle
20) Acide 2-hydroxy-4-[2-hydroxy-2-(3-tert-butyl-4-méthoxyphényl) éthoxy]benzoïque ;
21) Acide 2-hydroxy-4-[2-hydroxy-2-(3-tert-butyl-4-hydroxyphényl) éthoxy]benzoïque ;
22) Isomère (-) de l'acide 2-hydroxy-4-[2-hydroxy-2-(5,6,7,8-tétrahydro-5,5,8-8-tétraméthyl-2-naphtyl)éthoxy]benzoïque ;
23) Isomère (+) de l'acide 2-hydroxy-4-[2-hydroxy-2-(5,6,7,8-tétrahydro-5,5,8-8-tétraméthyl-2-naphtyl)éthoxy]benzoïque ;
24) Acide 2-hydroxy-4-[2-hydroxy-2(3-méthoxy-5,6,7,8-tétrahydro-5,5,8-8-tétraméthyl-2-naphtyl)éthoxy]benzoïque ;
25) Acide 2-méthoxy-4-[2-hydroxy-2-(5,6,7,8-tétrahydro-5,5,8-8-tétraméthyl-2-naphtyl)éthoxy]benzoïque ;
26) Acide 2-hydroxy-4-[2-hydroxy-2-(3-hydroxy-5,6,7,8-tétrahydro-5,5,8,8-tétraméthyl-2-naphtyl)éthoxy]benzoïque ;
27) Acide 2-hydroxy-4-[2-amino-2-(5,6,7,8-tétrahydro-5,5,8,8-tétraméthyl-2-naphtyl)éthoxy]benzoïque ;
28) Acide 2-hydroxy-4-[2-hydroxy-2-(5,6,7,8-tétrahydro-5,5,8,8-tétraméthyl-2-naphtyl)propyloxy]benzoïque ;
29) Acide 2-hydroxy-4-[2-hydroxy-2-(5,6,7,8-tétrahydro-5,5,8,8-tétraméthyl-2-naphtyl)hexyloxy]benzoïque ;
30) Acide 2-hydroxy-4-[2-hydroxy-2-(5,6,7,8-tétrahydro-5,5,8,8-tétraméthyl-2-naphtyl)éthylamino]benzoïque ;
31) Acide 2-hydroxy-4-[[2-hydroxy-2-[3-(1-adamantyl)-4-méthoxyphényl]éthoxy]]benzoïque ;
32) Acide 2-hydroxy-4-[[2-[3-(1-adamantyl)-4-méthoxyphenyl] éthoxy]]benzoïque.

La présente invention a également pour objet les procédés de préparation des composés des formules (Ia), (Ib), (Ic), (Id) et (Ie) selon les schémas réactionnels décrits ci-après : 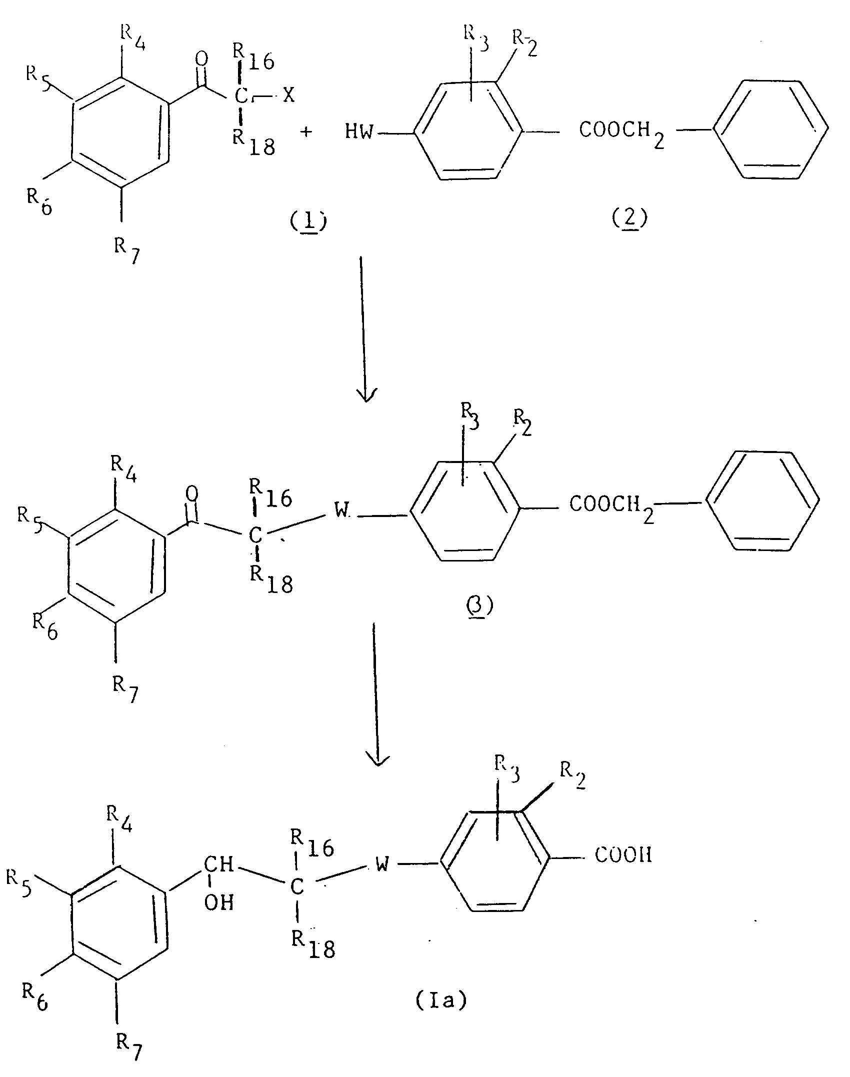
La première étape de cette préparation consiste à faire réagir en milieu anhydre dans un solvant organique tel que le DMF une -halocétone (1) avec un parahydroxy, paraamino ou parathiosalicylate de benzyle (2) en présence d'une amine tertiaire (pyridine ou triéthylamine) ou d'un hydrure alcalin (hydrure de sodium) pour obtenir le composé de formule (3).

L'étape principale consiste à hydrogéner le composé de formule (3) en présence d'un catalyseur tel que le palladium sur charbon dans un solvant organique tel que le dioxanne, le méthanol ou le THF. L'hydrogénation peut s'effectuer à une température entre 20 et 60°C sous une pression d'hydrogène comprise entre 1 bar et 7 bars et permet à la fois d'obtenir l'acide libre et de réduire la fonction cétonique.

Par action de l'hydroxylamine sur le composé (3), on obtient un hydroxymino. La réduction de l'hydroximino permet d'obtenir le composé aminé correspondant.

Les composés de formule générale (I) où X = (i) peuvent aussi être préparés par action d'un chlorure d'acide (5) avec un dérivé aromatique (4) en présence d'un acide Lewis (par exemple AlCl₃) dans un solvant chloré tel le dichlorométhane, le dichloroéthane, ou nitré tel le nitrométhane, le nitrobenzène. La cétone (6) ainsi obtenue est réduite en alcool avec un hydrure alcalin tel NaBH₄ dans un solvant organique tel que le THF, ou l'éthanol : 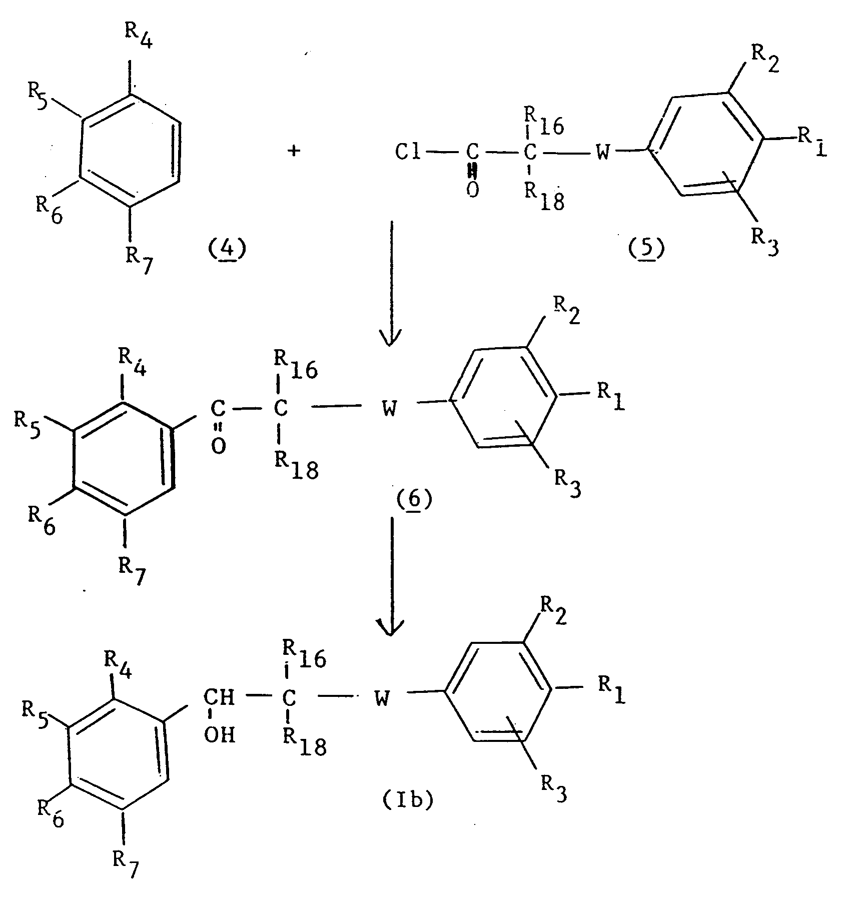
Les composés de formule générale (I) où X = (ii) peuvent être préparés par action d'un bromure de benzyle substitué (7) avec un alcool benzylique ou une amine benzylique ou un mercaptan benzylique substitué(e) (8) en présence de pyridine ou d'une amine tertiaire telle que la triéthylamine dans un solvant organique tel le DMF ou le THF, ou en présence d'un carbonate alcalin, tel le carbonate de potassium dans un solvant tel l'acétone ou la méthyléthylcétone. 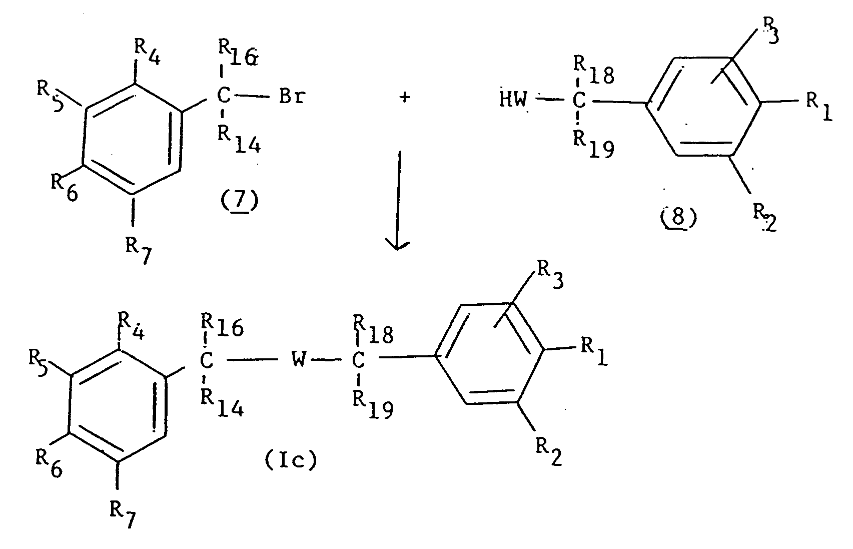
Les composés de formule générale (I) où X = (iv) peuvent être préparés par action d'une acétophénone substituée (9) avec un benzaldéhyde substitué (10) en présence d'une base telle la soude ou le méthylate de sodium dans un solvant alcoolique (éthanol). La chalcone (11) ainsi obtenue est réduite en alcool allylique (Id) à l'aide d'un hydrure alcalin tel NaBH₄ dans un solvant alcoolique en présence d'un catalyseur (CeCl₃).

Par hydrogénation du composé (Id) en présence d'un catalyseur tel le palladium sur charbon dans un solvant tel le dioxanne ou le méthanol on obtient des composés (Ie) de formule générale (I) où X = (iii). 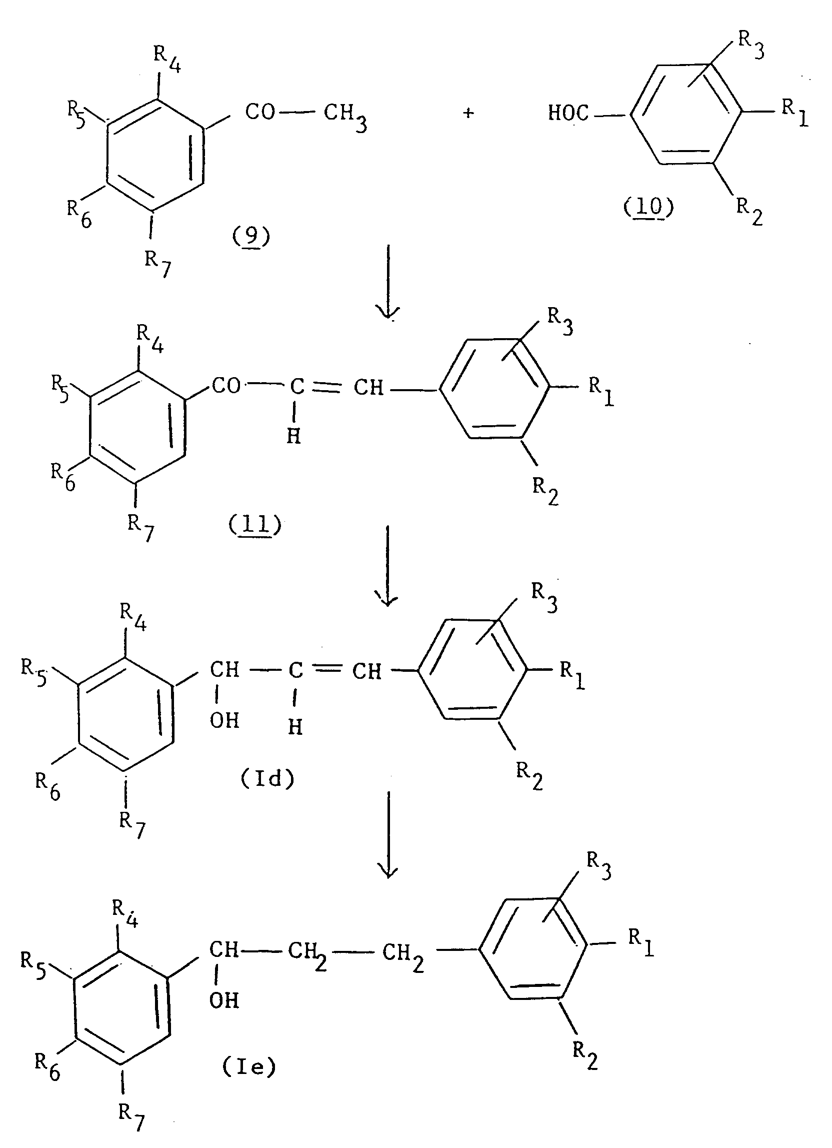
Par réaction de type Mitsunobu à partir des alcools (Ib), (Id) ou (Ie) on obtient le dérivé azido que l'on peut transformer en dérivé amino.

Par réaction d'un anhydride ou d'un chlorure d'acide sur le dérivé amino, on obtient l'amide correspondant.

Dans le cas où le radical X est lu de manière inversée par rapport aux schémas précédents aboutissant aux composés (Ia), (Ib), (Id) ou (Ie), les composés sont obtenus par les réactions décrites dans ces schémas en utilisant des produits de départ ayant des substituants appropriés.

Lorsque dans les composés selon l'invention X représente un radical di ou trihydroxylés ceux-ci sont obtenus par époxydation des composés éthyléniques correspondants et ouverture de la fonction époxy en milieu alcalin ou en présence d'un hydrure.

La présente invention a également pour objet à titre de médicament les composés de formule (I) tels que décrits ci-dessus.

Les composés selon l'invention présentent une bonne stabilité à la lumière et à l'oxygène.

Ces composés présentent une activité dans le test de différenciation des cellules (F9) de tératocarcinome embryonnaire de la souris (Cancer Research 43, p.5268, 1983) et/ou dans le test d'inhibition de l'ornithine décarboxylase après induction par le TPA chez la souris (Cancer Research 38, p.793-801, 1978) et/ou sur la différenciation des kératinocytes chez l'homme (Models Dermatol. Maibach HI, Lowe NJ Ed.Karger Bazel (1989) ou chez la rate (Pharmacol. Skin 1989 Vol.3 P. 141-143). Ces tests montrent les activités des composés dans les domaines de la différenciation et de la prolifération. Ces composés présentent en outre un bon index biologique.

Les composés selon l'invention conviennent particulièrement bien dans les domaines de traitements suivants :
1) Pour traiter les affections dermatologiques liées à un désordre de la kératinisation portant sur la différenciation et sur la prolifération notamment pour traiter les acnés vulgaires, comédoniennes, polymorphes, les acnés nodulokystiques, conglobata, les acnés séniles, les acnés secondaires telles que l'acné solaire, médicamenteuse, professionnelle.
2) Pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen, cutané ou muqueux (buccal).
3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation.
4) Pour traiter toutes les proliférations dermiques ou épidermiques qu'elles soient bénignes ou malignes, qu'elles soient d'origine virale telles que verrues vulgaires, les verrues planes et l'épidermodysplasie verruciforme, les papillomatoses orales ou florides et les proliférations pouvant également être induites par les ultra-violets notamment dans le cas des épithélioma baso et spinocellulaires.
5) Pour traiter d'autres désordres dermatologiques tels que les dermatoses bulleuses et les maladies du collagène.
6) Pour traiter certains troubles ophtalmologiques, notamment les cornéopathies.
7) Pour réparer ou lutter contre le vieillissement de la peau, qu'il soit photoinduit ou chronologique ou à réduire les pigmentations et les kératoses actiniques.
8) Pour prévenir ou guérir les stigmates de l' atrophie épidermique et/ou dermique induite par les corticostéroïdes locaux ou systémiques, ou tout autre forme d'atrophie cutanée.
9) Pour prévenir ou traiter les troubles de la cicatrisation ou pour prévenir ou réparer les vergétures.
10) Pour lutter contre les troubles de la fonction sébacée tels que l'hyperséborrhée de l'acné ou la séborrhée simple.
11) Dans le traitement d'états cancéreux en précancéreux en particulier au niveau cutané.
12) Dans le traitement d'affections inflammatoires telles que l'arthrite.

La présente invention a également pour objet des compositions médicamenteuses contenant au moins un composé de formule (I) telle que définie ci-dessus, ou un de ses sels.

La présente invention a donc aussi pour objet une nouvelle composition médicamenteuse destinée notamment au traitement des affections susmentionnées, caractérisée par le fait qu'elle comporte, dans un support pharmaceutiquement acceptable au moins un composé de formule (I) et/ou un de ses sels.

Les composés selon l'invention sont généralement administrés à une dose journalière d'environ 0,01 mg/kg à 100 mg/kg en poids corporel en 1 à 3 prises.

L'administration peut être effectuée par voie entérale, parentérale, topique ou oculaire. Par voie entérale, les médicaments peuvent se présenter sous forme de comprimés, de gelules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée.

Ces compositions par voie topique peuvent se présenter soit sous forme anhydre, soit sous forme aqueuse selon l'indication clinique.

Par voie oculaire, ce sont principalement des collyres.

Ces compositions contiennent au moins un composé de formule (I) telle que définie ci-dessus ou un de ses sels, à une concentration de préférence comprise entre 0,001 et 5 % par rapport au poids total de la composition.

Les composés de formule (I), selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention vise donc également une composition cosmétique contenant, dans un support cosmétiquement acceptable, au moins un composé de formule (I) ou un de ses sels, cette composition se présentant notamment sous forme d'une crème, d'un lait, d'une lotion, d'un gel, de microsphères ou nanosphères ou vésicules lipidiques ou polymériques, d'un savon ou d'un shampooing.

La concentration en composé de formule (I), dans les compositions cosmétiques est comprise entre 0,001 et 3 % en poids.

Les compositions médicamenteuses et cosmétiques selon l'invention peuvent contenir des additifs inertes ou même pharmacodynamiquement ou cosmétiquement actifs ou des combinaisons de ceux-ci et notamment : des agents mouillants, des agents dépigmentants tels que l'hydroquinone, l'acide azélaïque, l'acide caféïque ou l'acide kojique, des émollients, des agents hydratants comme le glycérol, le PEG 400, la thiamorpholinone et ses dérivés ou l'urée ; des agents antiséborrhéiques ou antiacnéiques, tels que la S-carboxyméthylcystéine, la S-benzyl-cystéamine, leurs sels et leurs dérivés, la tioxolone ou le peroxyde de benzoyle ; des antibiotiques comme l'érythromycine et ses esters, la néomycine, la clindamycine et ses esters, les tétracyclines, des agents antifongiques tels que le kétoconazole ou les polyméthylène-4,5 isothiazolinones-3 ; des agents favorisant la repousse des cheveux, comme le "Minoxidil" (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,5-diphényl-imidazolidine 2,4-dione) ; des agents anti-inflammatoires stéroïdiens et non stéroïdiens; des caroténoïdes et, notamment le β-carotène ; des agents anti-psoriatiques tels que l'anthraline et ses dérivés et les acides eicosa-5,8,11,14-tétraynoïque et eicosa-5,8,11-trynoïque, leurs esters et les amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants des filtres UV-A et UV-B, des anti-oxydants tels que l'α-tocophérol, le butylhydroxyanisole ou le butylhydroxytoluène.

On va maintenant donner, à titre d'illustration et sans aucun caractère limitatif, plusieurs exemples de préparation des composés actifs de formule (I) selon l'invention ainsi que des exemples de compositions les contenant.

### A. EXEMPLES DE COMPOSES

### EXEMPLE 1

### Acide 2-hydroxy-4-[2-hydroxy-2-(5,6,7,8-tétrahydro-5,5,8,8-tétraméthyl-2-naphtyl)éthoxy]benzoîque.

### (a) 2,4-dihydroxybenzoate de benzyle :

A une solution de 3 g (0,1 mole) d'hydrure de sodium (80 % dans l'huile) et 50 ml de DMF, on ajoute goutte à goutte 15,4 g (0,1 mole) d'acide 2,4-dihydroxybenzoïque dissous dans 50 ml de DMF et agite à température ambiante jusqu'à cessation du dégagement gazeux. On ajoute ensuite 13,1 ml (0,1 mole) de bromure de benzyle et agite à température ambiante jusqu'à solubilisation du milieu réactionnel. On verse le mélange réactionnel dans l'eau, extrait avec de l'éther éthylique, décante la phase organique, lave à l'eau, sèche sur sulfate de magnésium et évapore. Le résidu est purifié par chromatographie sur colonne de silice en éluant avec du dichlorométhane. On recueille 19,7 g (81 %) de l'ester attendu, qui fond à 94-95°C.

### (b) 2-(2'-bromoacétyl)-5,6,7,8-tétrahydro-5,5,8,8-tétraméthylnaphtalène :

Dans un ballon on introduit 3,5 g (15,2 mmoles) de 2-acétyl-5,6,7,8-tétrahydro-5,5,8,8-tétraméthylnaphtalène, 25 ml d'éther éthylique et 25 ml de dioxanne. On ajoute goutte à goutte 810 µl (15,2 mmoles) de brome et agite à température ambiante pendant une heure. On verse le milieu réactionnel dans l'eau, extrait avec de l'éther éthylique, décante la phase organique, sèche sur sulfate de magnésium et évapore. Le résidu obtenu est purifié par chromatographie sur colonne de silice, élué avec un mélange de dichlorométhane et d'hexane (30-70). Après évaporation des solvants, on recueille 3,5 g (74 %) de dérivé bromé, sous forme de cristaux lègèrement jaunes, fondant à 61-62°C.

### (c) 2-hydroxy-4-(5,6,7,8-tétrahydro-5,5,8,8-tétraméthyl-2-naphtoylméthoxy)benzoate de benzyle :

Dans un ballon, on introduit 300 mg (10 mmoles) d'hydrure de sodium (80 % dans l'huile) et 25 ml de DMF. On ajoute goutte à goutte une solution de 2,4 g (10 mmoles) de 2,4-dihydroxybenzoate de benzyle dans 75 ml de DMF et agite jusqu'à cessation du dégagement gazeux. On introduit ensuite une solution de 3,1 g (10 mmoles) du dérivé bromé préparé précédemment dans 50 ml de DMF et agite à température ambiante pendant 2 heures. On verse le milieu réactionnel dans l'eau, extrait avec de l'éther éthylique, décante la phase organique, sèche sur sulfate de magnésium et évapore. Le résidu obtenu est purifié par chromatographie sur colonne de silice élué avec mélange de dichlorométhane et d'hexane (50-50). Après évaporation des solvants, on recueille 3,4 g (73 %) du produit attendu qui fond à 103-104°C.

### (d) Acide 2-hydroxy-4-[2-hydroxy-2-(5,6,7,8-tétrahydro-5,5,8,8-tétraméthyl-]2-naphtyl)éthoxy benzoîque :

Dans un réacteur, on introduit 2,9 g (6,1 mmoles) de l'ester préparé précédemment, 1 g de palladium sur charbon (10 %) et 100 ml de dioxanne. On hydrogène à température ambiante et sous une pression de 7 bars pendant 4 heures, filtre le catalyseur, lave avec 2 fois 50 ml de THF, et évapore les filtrats. Le résidu obtenu est purifié par chromatographie sur colonne de silice en éluant avec un mélange de dichlorométhane et d'éther éthylique (95-5). Après évaporation des solvants, on recueille 2 g (87 %) d'acide 2-hydroxy-4- 2-hydroxy-2-(5,6,7,8-tétrahydro-5,5,8,8-tétraméthyl-2-naphtyl)éthoxy benzoïque qui fond à 206-207°C.

### EXEMPLE 2

### 2-hydroxy-4-[2-hydroxy-2-(5,6,7,8-tétrahydro-5,5,8,8-tétraméthyl-2-naphtyl)éthoxy]benzoate de méthyle.

### (a) 2-hydroxy-4-(5,6,7,8-tétrahydro-5,5,8,8-tétraméthyl-2-naphtoylméthoxy)benzoate de méthyle :

Dans un ballon on introduit 990 mg (33 mmoles) d'hydrure de sodium (80 % dans l'huile) et 50 ml de DMF. Sous courant d'azote, on ajoute goutte à goutte une solution de 5,6 g (33 mmoles) de 2,4-dihydroxybenzoate de méthyle dans 50 ml de DMF et agite jusqu'à cessation de dégagement gazeux. On introduit ensuite goutte à goutte une solution de 9,4 g (33 mmoles) de bromocétone préparée en 1(b) dissous dans 75 ml de DMF et agite à température ambiante 2 heures. On verse le milieu réactionnel dans l'eau, extrait avec de l'éther éthylique, décante la phase organique, sèche sur sulfate de magnésium et évapore. Le résidu obtenu est purifié par chromatographie sur colonne de silice, élué avec un mélange de dichlorométhane et d'hexane (70-30). Après évaporation des solvants, on recueille 8,5 g (72 %) de l'ester attendu qui fond à 113-114°C.

### (b) 2-hydroxy-4-[2-hydroxy-2-(5,6,7,8-tétrahydro-5,5,8,8-tétraméthyl-2-naphtyl)éthoxy]benzoate de méthyle :

Dans un ballon, on introduit 1,6 g (4 mmoles) de la cétone préparée précédemment, 50 ml de THF et 50 ml de méthanol. On ajoute par petites quantités 80 mg (2 mmoles) de borohydrure de sodium et agite à température ambiante 2 heures. On évapore à sec le milieu réactionnel, reprend par de l'eau et de l'éther éthylique, décante la phase organique, sèche sur sulfate de magnésium et évapore. On triture le solide obtenu dans l'hexane, filtre, sèche sous vide. On recueille 1,6 g (100 %) du produit attendu qui fond à 133-134°C.

### EXEMPLE 3

### Acide 2-hydroxy-4-[2-hydroxyimino-2-(5,6,7,8-tétrahydro-5,5,8,8-tétraméthyl-2-naphtyl)éthoxy]benzoïque

### (a) 2-hydroxy-4-[2-hydroxyimino-2-(5,6,7,8-tétrahydro-5,5,8,8-tétraméthyl-2-naphtyl)éthoxy] benzoate de méthyle.

Dans un ballon, on introduit 6,6 g (16,6 mmoles) de 2-hydroxy-4-(5,6,7,8-tétrahydro-5,5,8,8-tétraméthyl-2-naphtoylméthoxy) benzoate de méthyle, 200 ml d'éthanol et 4,6 g (66,6 mmoles) de chlorhydrate d'hydroxylamine. On ajoute goutte à goutte 66 ml d'hydroxyde de sodium (1 N) et chauffe à reflux pendant 2 heures. On évapore à sec, reprend le résidu par de l'eau et de l'éther éthylique, décante la phase organique, sèche sur sulfate de magnésium et évapore. Le résidu obtenu est purifié par chromatographie sur colonne de silice, élué avec du dichlorométhane. Après évaporation des solvants, on obtient 4,4 g (64 %) de l'isomère syn qui fond à 138-9°C et 1,9 g (30 %) de l'isomère anti qui fond à 165-166°C.

### (b) Acide 2-hydroxy-4-[2-hydroxyimino-2-(5,6,7,8-tétrahydro-5,5,8,8-tétraméthyl-2-naphtyl)éthoxy]benzoïque :

Dans un ballon, on introduit 2,05 g (5 mmoles) de l'isomère syn précédent, 50 ml de THF et 50 ml de soude méthanolique 2 N. On chauffe à reflux 8 heures, évapore le milieu réactionnel, reprend le résidu par l'eau, neutralise avec de l'acide chlorhydrique concentré et extrait avec de l'éther éthylique. On décante la phase organique, sèche sur sulfate de magnésium, évapore. Le résidu obtenu est trituré dans le dichlorométhane et filtré. On obtient 1,6 g (81 %) du produit attendu qui fond à 220-222°C en se décomposant.

### EXEMPLE 4

### Acide 2-acétyloxy-4-[2-acétyloxy-2-(5,6,7,8-tétrahydro-5,5,8,8-tétraméthyl-2-naphtyl)éthoxy]benzoïque

### (a) 2-hydroxy-4-[2-hydroxy-2-(5,6,7,8-tétrahydro-5,5,8,8-tétraméthyl-2-naphtyl)éthoxy] benzoate de benzyle.

Dans un ballon, on introduit 5 g (10,5 mmoles) de 2-hydroxy-4-(5,6,7,8-tétrahydro-5,5,8,8-tétraméthyl-2-naphtoylméthoxy)benzoate de benzyle, 50 ml de THF et 50 ml de méthanol. On ajoute par petites quantités 200 mg (5,3 mmoles) de borohydrure de sodium et agite à température ambiante pendant 1 heure. On évapore à sec le milieu réactionnel, reprend par de l'eau et de l'éther éthylique, décante la phase organique, sèche sur sulfate de magnésium et évapore. On recueille 5 g (100 %) du produit attendu sous forme d'une huile légèrement jaune.

### (b) 2-acétyloxy-4-[2-acétyloxy-2-(5,6,7,8-tétrahydro-5,5,8,8-tétraméthyl-2-naphtyl)éthoxy]benzoate de benzyle ;

Dans un ballon, on introduit 4,8 g (10 mmoles) du produit précédent, 50 ml de THF et 4,2 ml (30 mmoles) de triéthylamine. On ajoute goutte à goutte 2,2 ml (30 mmoles) de chlorure d'acétyle et agite à température ambiante 8 heures. On verse le milieu réactionnel dans l'eau, extrait avec de l'éther éthylique, décante la phase organique, sèche sur sulfate de magnésium et évapore. Le résidu obtenu est purifié par chromatographie sur colonne de silice, élué avec du dichlorométhane. Après évaporation des solvants, on recueille 3,8 g (76 %) du produit attendu, sous forme d'une huile.

### (c) Acide 2-acétyloxy-4-[2-acétyloxy-2-(5,6,7,8-tétrahydro-5,5,8,8-tétraméthyl-2-naphtyl)éthoxy]benzoïque :

Dans un réacteur, on introduit 1,5 g (2,7 mmoles) du produit précédent, 200 ml de dioxanne et 300 mg de Pd/C (10 %). On hydrogène à température ambiante et sous une pression de 7 bars pendant 2 heures. On filtre le catalyseur, lave avec 2 fois 50 ml de THF, évapore les filtrats. Le résidu obtenu est trituré dans un mélange d'hexane et d'éther éthylique (90-10), filtré, séché sous vide. On recueille 1,2 g (92 %) du produit attendu, qui fond à 88-89°C.

### EXEMPLE 5

### Acide-2-hydroxy-4-[2-acétyloxy-2-(5,6,7,8-tétrahydro-5,5,8,8-tétraméthyl-2-naphtyl)éthoxy]benzoïque

### (a) 2-hydroxy-4-[2-acétyloxy-(5,6,7,8-tétrahydro-5,5,8,8-tétraméthyl-2-naphtyl)éthoxy] benzoate de benzyle

Dans un ballon, on introduit 2,2 g (4,6 mmoles) de 2-hydroxy-4-[2-hydroxy-2-(5,6,7,8-tétrahydro-5,5,8,8-tétraméthyl-2-naphtyl)éthoxy]benzoate de benzyle, 50 ml de THF et 380 µl (4,6 mmoles) de pyridine. On ajoute goutte à goutte 330 µl (4,6 mmoles) de chlorure d'acétyle et agite à température ambiante pendant 8 heures. On verse le milieu réactionnel dans l'eau, extrait avec de l'éther éthylique, décante la phase organique, sèche sur sulfate de magnésium et évapore. Le résidu obtenu est purifié par chromatographie sur colonne de silice, élué avec un mélange d'hexane et de dichlorométhane (90-10). Après évaporation des solvants, on recueille 1,8 g (77 %) du produit attendu sous forme d'une huile jaune.

### (b) Acide 2-hydroxy-4-[2-acétyloxy-2-(5,6,7,8-tétrahydro-5,5,8,8-tétraméthyl-2-naphtyl)éthoxy]benzoïque

De manière analogue à l'exemple 4(c) à partir de 1,5 g (2,9 mmoles) du produit précédent, on obtient 1,1 g (90 %) du produit attendu qui fond à 160-161°C.

### EXEMPLE 6

### Acide 2-acétyloxy-4-[2-hydroxy-2-(5,6,7,8-tétrahydro-5,5,8,8-tétraméthyl-2-naphtyl)éthoxy]benzoïque

### (a) 2-acétyloxy-4-(5,6,7,8-tétrahydro-5,5,8,8-tétraméthyl-2-naphtoylméthoxy)benzoate de benzyle

De manière analogue à l'exemple 4(b) par réaction de 2,36 g (5 mmoles) de 2-hydroxy-4-(5,6,7,8-tétrahydro-5,5,8,8-tétraméthyl-2-naphtoylméthoxy)benzoate de benzyle et 360 µl (5 mmoles) de chlorure d'acétyle, on recueille 2 g (80 %) du produit attendu qui fond à 137-138°C.

### (b) Acide 2-acétyloxy-4-[2-hydroxy-2-(5,6,7,8-tétrahydro-5,5,8,8-tétraméthyl-2-naphtyl)éthoxy]benzoïque

De manière analogue à l'exemple 1(d) à partir de 1,9 g (3,7 mmoles) du produit précédent, on obtient 1,4 g (89 %) du produit attendu qui fond à 119-120°C.

### EXEMPLE 7

### Acide-2-hydroxy-4-[2-(5,6,7,8-tétrahydro-5,5,8,8-tétraméthyl-2-naphtyl)éthoxy]benzoïque

Dans un réacteur, on introduit 2g (3,89 mmoles) de 2-hydroxy-4-[2-acétyloxy-2-(5,6,7,8 -tétrahydro-5,5,8,8-tétraméthyl-2-naphtyl)éthoxy]benzoate de benzyle obtenu à l'exemple 5(a), 100 ml d'éthanol, 10 ml d'acide acétique et 1,2 g de palladium sur charbon (10% à 50% d'eau). On hydrogène à 70°C, sous une pression de 7 bars pendant 4 heures. On filtre le catalyseur, lave avec l'éthanol, évapore le filtrat. Le résidu obtenu est repris dans l'hexane, filtré et séché. On recueille 650 mg (46%) du produit attendu, de point de fusion 210-212 °C.

### EXEMPLE 8

### 2-hydroxy-4-[2-hydroxy-2-(5,6,7,8-tétrahydro-5,5,8,8-tétraméthyl-2-naphtyl]ethoxy]benzyl alcool

Dans un ballon, on introduit 1,27 g (2,68 mmoles) de 2-hydroxy-4-[2-hydroxy-2-(5,6,7,8-tétrahydro-5,5,8,8-tétraméthyl-2-naphryl)éthoxy]benzoate de benzyle, 15 ml de THF. On ajoute par petites quantités 480 mg (12 mmoles) de LiAlH4 (96%). On agite à température ambiante pendant 15 minutes. On ajoute par petites quantités Na2SO4 hydraté. On laisse agiter à température ambiante pendant une nuit. On filtre l'insoluble, lave par THF et évapore. Le résidu obtenu est purifié par chromatographie sur colonne de silice, éluée avec un mélange hexane-acétate d'éthyle (60/40). Après évaporation des solvants, on recueille 260 mg (26%) du produit attendu, sous forme d'une huile qui cristallise lentement à température ambiante, de point de fusion 110-115°C.

### EXEMPLE 9

### Acétate de 2-acétyloxy-4-[2-hydroxy-2-(5,6,7,8-tétrahydro-5,5,8,8-tétraméthyl-2-naphtyl) éthoxy]benzyl alcool

Dans un ballon, on introduit 1,2 g (3,23 mmoles) de 2-hydroxy4-[2-hydroxy-2-(5,6,7,8-tétrahydro-5,5,8,8-tétraméthyl-2-naphryl)éthoxy]benzyl alcool, 30 ml de pyridine et ajoute 345 ml (4,84 mmoles) de chlorure d'acétyle. On agite à 0°C pendant 4 heures. On verse le milieu réactionnel dans l'eau, acidifie avec de l'acide chlorhydrique, extrait avec de l'acétate d'éthyle, lave à l'eau, décante la phase organique, sèche sur sulfate de sodium et évapore. Le résidu obtenu est purifié par chromatographie sur colonne de silice, éluée avec un mélange d'hexane et d'acétate d'éthyle (70/30). Après évaporation des solvants, on recueille 140 mg (10,4%) du produit attendu sous forme d'une huile jaune pâle.

### EXEMPLE 10

### Pipéridinylamide de l'acide 2-hydroxy-4-[2-hydroxy-2-(5,6,7,8-tétrahydro-5,5,8,8-tétraméthyl -2-naphtyl)éthoxy]benzoïque

Dans un ballon, on introduit 3,43 g (7,6 mmoles) de pipéridinylamide de l'acide 2-hydroxy-4-(5,6,7,8-tétrahyo-5,5,8,8-tétraméthyl-2-naphtoylméthoxy)benzoïque, 100ml d'isopropanol et 50 ml de THF. On refroidit à 0°C, ajoute 144 mg (3,81 mmoles) de NaBH4. On agite à 0°C pendant 1 heure. On ajoute de l'acétone, évapore les solvants, reprend dans l'eau, ajuste à pH 6-7 avec de l'acide chlorhydrique (1N). On extrait par l'acétate d'éthyle, lave à l'eau, sèche sur sulfate de sodium et évapore les solvants. Le résidu obtenu est purifié par chromatographie sur colonne de silice éluée avec un mélange d'acétate d'éthyle et d'hexane (35/65). Après évaporation des solvants, on cristalise l'huile otenu dans l'hexane, filtre et sèche, on recueille 4,25 g (86%) de produit attendu, de point de fusion 124°C.

### EXEMPLE 11

### Morpholinylamide de l'acide 2-hydroxy-4-[2-hydroxy-2-(5,6,7,8-tétrahydro-5,5,8,8-tétraméthyl-2-naphtyl)éthoxy]benzoïque

De manière analogue à l'exemple 10, à partir 5,5 g (12 mmoles) de morpholinylamide de l'acide 2-hydroxy-4-(5,6,7,8-tétrahydro-5,5,8,8-tétraméthyl-2-naphtoylméthoxy)benzoïque et par recristallisation dans 10 volumes d'éthanol. On recueille 4,24g (77%) du produit attendu, de point de fusion 153°C.

### EXEMPLE 12

### 2-hydroxy-4-[2-hydroxy-2-(5,6,7,8-tétrahydro-5,5,8,8-tétraméthyl-2-nahptyl)éthoxy] benzamide

De manière analogue à l'exemple 10, à partir de 4,4 g (11 mmoles) de 2-hydroxy-4-(5,6,7,8-tétrahydro-5,5,8,8-tétraméthyl-2-naphtoylméthoxy)benzamide, on obtient une huile jaune qui cristallise dans un mélange éthanol/eau. Après filtration et séchage, on recueille 3,72 g (84%) du produit attendu, de point de fusion 85-90°C.

### EXEMPLE 13

### N-éthyl amide de l'acide 2-hydroxy-4-[2-hydroxy-2-(5,6,7,8-tétrahydro-5,5,8,8-tétraméthyl -2-nahptyl)éthoxy]benzoïque

De manière analogue à l'exemple 10, à partir de 2,4 g (5,87 mmoles) de N-éthyl amide de l'acide 2-hydroxy-4-(5,6,7,8-tétrahydro-5,5,8,8-tétraméthyl-2-nahptoylméthoxy)benzoïque, on obtient une huile jaune que l'on cristallise dans l'hexane. Après filtration et séchage, on recueille 2,24g (93%) du produit attendu, de point de fusion 65-70°C.

### EXEMPLE 14

### 2-hydroxy-4-[2-hydroxy-2-(4,4-diméthylthiochroman-6-yl)éthoxy]benzoate de méthyle

### a) 6-(2-bromoacétyl)-4,4-diméthylthiochromane

De manière analogue à l'exemple 1(b), on réalise la synthèse à partir de 1 g (4,42 mmoles) de 6-acétyl-4,4-diméthylthichroman et 700 mg (4,42 mmoles) de brome. Après traitement et purification par chromatographie sur colonne de silice, en éluant avec un mélange dichlorométhane-hexane (40/60), on obtient 700 mg (53%) de dérivé bromé attendu, sous forme d'une huile marron.

### b) 2-hydroxy-4-(4,4-diméthylthiochroman-6-oylméthoxy)benzoate de méthyle

Dans un tricol de 100 ml sous azote, on verse 5 ml de diméthylformamide et 80 mg (2,75 mmoles) d'hydrure de sodium à 80%. On introduit goutte-à-goutte et à température ambiante 430 mg (2,57 mmoles) de 2,4-dihydroxybenzoate de méthyle dissous dans 20 ml de diméthylformamide et agite jusqu'à cessation de dégagement d'hydrogène. On ajoute ensuite 770 mg (2,57 mmoles) de dérivé bromé obtenu précédemment, issous dans 15 ml de diméthylformamide. On laisse agiter à température ambiante pendant 5 heures, verse dans l'eau, extrait à l'éther éthylique et sèche sur sulfate de sodium. Après filtration, on évapore les solvants et récupère 1 g de produit brut que l'on chromatographie sur colonne de silice en éluant avec du dichlorométhane. On obtient ainsi 540 mg (53%) de produit attendu, de point de fusion 135-137°C.

### c) 2-hydroxy-4-[2-hydroxy-(4,4-diméthylthiochroman-6-yl)éthoxy]benzoate de méthyle

Dans un tricol de 50 ml sous azote, on introduit 540 mg (1,4 mmole) du dérivé obtenu précédemment, dissous dans 15 ml de THF. On ajoute 50 mg (1,4 mmole) de borohydrure de sodium. Après 30 minutes de réaction à température ambiante, on verse le milieu réactionnel dans l'eau et extrait par l'acétate d'éthyle, sèche sur sulfate de sodium, filtre et évapore à sec la phase organique. On obtient 530 mg de produit attendu, cristallisant dans l'hexane, de point de fusion 113-115°C.

### EXEMPLE 15

### Acide 2-hydroxy-4[2-hydroxy-2-(4,4-diméthylthiochroman-6-yl)éthoxy]benzoïque

Dans un bailon de 100 ml, on introduit 450 mg (1,16 mmole) du dérivé précédent, 10 ml de méthanol et ajoute 460 mg (11,6 mmoles) de soude en pastille. On chauffe à reflux durant 12 heures, évapore le solvant et reprend dans l'eau. On acidifie, extrait par l'acétate d'éthyle, sèche la phase organique sur sulfate de sodium, filtre et évapore à sec. On récupère 500 mg d'une huile marron qui est chromatographée sur colonne de slice en éluant avec l'acétate d'éthyle. Après évaporation du solvant, le résidu est trituré dans l'hexane et filtré. On obtient ainsi 210 mg (49%) d'acide attendu, de point de fusion 166-167°C.

### EXEMPLE 16

### Acide 2-hydroxy-4-[3-hydroxy-3-(5,6,7,8,-tétrahydro-5,5,8,8-tétraméthyl-2-naphtyl) propyl]benzoïque

### a) Acide 2-hydroxy-4-[3-oxo-3-(5,6,7,8,-tétrahydro-5,5,8,8-tétraméthyl-2-naphyl)1-propényl]benzoïque

Dans un ballon, on introduit 2,3 g (10 mmoles) de 2-acéto-5,6,7,7,8-tétrahydro-5,5,8,8-tétramétylnaphtone, 1,8 g (10 mmoles) de 2-hydroxy-4-formylbenzoate de méthyle, 70 ml de méthanol et 40 ml d'hydroxyde de sodium (1N). On agite à température ambiante pendant 24 heures, évapore à sec, reprend par eau, acidifie avec de l'acide chlorhydrique, extrait avec de l'éther éthylique, décante la phase organique, sèche sur sulfate de magnésium, évapore. Le résidu est recristallisé dans l'alcool éthylique, filtré, séché. On recueille 1,5 g (41%) du produit attendu, de point de fusion 260-261°C.

### b) Acide 2-hydroxy-4-[3-hydroxy-3-(5,6,7,8,-tétrahydro-5,5,8,8-tétraméthyl-2-naphtyl) propyl]benzoïque

On hydrogène, à température ambiante et sous une pression de 7 bars 1,5 g (4 mmoles) de l'acide précédent dans 60 ml de dioxanne en présence de 550 mg de palladium sur charbon à 10% pendant 4 heures. Après filtration et évaporation du filtrat, on triture le résidu obtenu dans l'hexane et filtre. On recueille 870 mg (57%) du produit attendu, de point de fusion 144-145°C.

### EXEMPLE 17

### Acide 2-hydroxy-4-[2-hydroxy-2-(3,5-di-tert-butyl-4-hydroxyphényl)éthoxy]benzoïque

### a) 3,5-di-tert-butyl-4-hydroxy-(2'-bromoacéto)phénone

De manière analogue à l'exemple 1(b), à partir de 2,5 g (10 mmoles) de 3,5-di-tert-butyl-4-hydroxy-acétophénone on recueille 1,6 g (48%) de dérivé bromé sous forme d'une huile légèrement jaune.

### b) 2-hydroxy-4-(3,5-di-tert-butyl-4-hydroxybenzoylméthoxy)benzoate de benzyle

De manière analogue à l'exemple 19(a), par réaction de 1,6 g (4,9 mmoles) du dérivé bromé précédent avec 1,2 g (4,9 mmoles) de 2,4-dihydroxybenzoate de benzyle, on obtient 2 g (83%) de l'ester attendu, de point de fusion 122-123°C.

### c) Acide 2-hydroxy-4-[(3,5-di-tert-butyl-4-hydroxybenzoyl)méthoxy]benzoïque

Dans un réacteur, on introduit 1,5 g (3,06 mmoles) de l'ester précédent, 60 ml de dioxanne et 300 mg de palladium sur charbon à 10%. On hydrogène à température ambiante et sous une pression de 7 bars durant 1 heure, filtre le catalyseur, évapore le filtrant. Le résidu obtenu est trituré dans l'hexane, filtré, séché. On recueille 1 g (82%) du produit attendu, de point de fusion 164-165°C.

### d) Acide 2-hydroxy-4-[2-hydroxy-2-(3,5-di-tert-butyl-4-hydroxyphényl)éthoxy]benzoïque

De manière analogue à l'exemple 2(b), à partir de 1 g (2,5 mmoles) de l'ester précédent, on obtient 710 mg (71%) du produit attendu, de point de fusion 132-133°C.

### EXEMPLE 18

### 2-hydroxy-4-[2-hydroxy-2-(5,6,7,8-tétrahydro-5,5,8,8-tétraméthyl-2-naphtyl)éthoxy]toluène

### a) 2-hydroxy-4-(5,6,7,8-tétrahydro-5,5,8,8-tétraméthyl-2-naphtoylméthoxy)benzaldéhyde

De manière analogue à l'exemple 19(a), par réaction de 6,2 g (20 mmoles) de 2-(2'-bromoacéto)-5,6,7,8-tétrahydro-5,5,8,8-tétraméthylnaphtone avec 2,8 g (20 mmoles) de 2,4-dihydroxybenzaldéhyde, on obtient 6,9 g (94%) d'aldehyde attendu, sous forme d'une huile incolore.

### b) 2-hydroxy-4-[2-hydroxy-2-(5,6,7,8-tétrahydro-5,5,8,8-tétraméthyl-2-naphtyl) éthoxy]toluène

On hydrogène à température ambiante et sous une pression de 7 bars, 1 g (2,7 mmoles) de l'aldéhyde précédent, en présence de 200 mg de palladium sur charbon à 10%. Après filtration et évaporation du filtrat, on purifie le résidu obtenu par chromatographie sur colonne de silice, en éluant avec un mélange éther éthylique-hexane (30/70). On recueille 600 mg (62%) du produit attendu, de point de fusion 120-121°C.

### EXEMPLE 19

### 2,6-dihydroxy-4-[2-hydroxy-2-(5,6,7,8-tétrahydro-5,5,8,8-tétraméthyl-2-naphtyl)éthoxy] benzoate de méthyle

### a) 2,6-dihydroxy-4-(5,6,7,8-tétrahydro-5,5,8,8-tétraméthyl-2-naphtoylméthoxy)benzoate de méthyle

Dans un ballon, on introduit 3,1 g (10 mmoles) de 2-(2'-bromoacéto)-(5,6,7,8-tétrahydro-5,5,8,8-tétraméthylnaphtone, 1,8 g (10 mmoles) de 2,4,6-trihydroxybenzoate de méthyle, 1,4 g (10 mmoles) de carbonate de potassium et 100 ml de méthyléthylcétone. On chauffe à reflux durant 1 heure et évapore à sec. On reprend le résidu par eau et dichlorométhane, décante la phase organique, sèche sur sulfate de magnésium, évapore. Le résidu obtenu est purifié par chromatographie sur colonne de silice, éluée avec un mélange de dichlorométhane et d'hexane(50/50). Après évaporation des solvants, on recueille 2,2 g (53%) du produit attendu, de point de fusion 169-170°C.

### b) 2,6-dihydroxy-4-[2-hydroxy-2-(5,6,7,8-tétrahydro-5,5,8,8-tétraméthyl-2-naphtyl)éthoxy] benzoate de méthyle

De manière analogue à l'exemple 2(b), à partir de 2,7 g (6,5 mmoles) de l'ester précédent, on obtient 2,1 g (77%) du produit attendu, de point de fusion 127-128°C.

### EXEMPLE 20

### Acide 2-hydroxy-4-[2-hydroxy-2-(3-tert-butyl-4-méthoxyphényl)éthoxy]benzoïque

### a) 3-tert-butyl-4-méthoxyacétophénone

Dans un tricol et sous courant d'azote, on introduit 22,6 g (0,1 mole) de chlorure de 3-tert-butyl-4-méthoxybenzoyle, 30 ml de HMPA, 14 ml (0,1 mole) de tétraméthylétain et 43 mg de benzyl(chloro)bis(triphénylphosphine)palladium(II). On chauffe à 80°C durant 4 heures, verse dans l'eau, extrait avec de l'éther éthylique, décante la phase organique, sèche sur sulfate de magénsium, évapore. On purifie le résidu obtenu par chromatographie sur colonne de silice éluée, avec un mélange dichlorométhane-hexane (50/50). Après évaporation des solvants, on recueille 11,5 g (58%) de la cétone attendue, de point de fusion 68-69°C.

### b) 3-tert-butyl-4-méthoxy-(2-bromo)acétophénone

De manière analogue à l'exemple 1(b), à partir de 8,24 g (40 mmoles) de la cétone obtenue en 20(a), on obtient 8,7 g (76%) de dérivé bromé, sous forme d'une huile légèrement jaune.

### c) 2-hydroxy-4-[(3-tert-butyl-4-méthoxybenzoyl)méthoxy]benzoate de benzyle

De manière analogue à l'exemple 19(a), par réaction de 8,7 g (30 mmoles) du dérivé bromé précédent, avec 7,5 g (30 mmoles) de 2,4-dihydroxybenzoate de benzyle, on obtient 11 g (80%) de l'ester attendu, de point de fusion 98-99°C.

### d) Acide 2-hydroxy-4-[2-hydroxy-2-(3-tert-butyl-4-méthoxyphényl)éthoxy]benzoïque

De manière analogue à l'exemple 1(d), à partir de 5 g (11,2 mmoles) de 2-hydroxy-4-(3-tert-butyl-4-méthoxybenzoylméthoxy)benzoate d'allyle, on obtient 4 g (99%) d'acide attendu, de point de fusion 149-150°C.

### EXEMPLE 21

### Acide 2-hydroxy-4-[2-hyroxy-2-(3-tert-butyl-4-hydroxyphényl)éthoxy]benzoïque

### a) 3-tert-butyl-4-benzyloxybenzoate de méthyle

De manière analogue à l'exemple 1(a), par réaction de 17 g (82 mmoles) de 3-tert-butyl-4-hydroxybenzoate de méthyle, avec 10,7 ml (82 mmoles) de bromure de benzyle, on obtient 24,4 g (100%) du produit attendu, sous forme d'une huile incolore.

### b) Acide 3-tert-butyl-4-benzyloxybenzoïque

Dans un ballon, on introduit 24,4 g (82 mmoles) de l'ester précédent et 400 ml de soude méthanolique 1N. On chauffe à reflux 3 heures, évapore à sec, reprend par eau, acidifié à pH=1, extrait avec de l'éther éthylique, décante la phase organique, sèche sur sulfate de magnésium, évapore. Le résidu obtenu est trituré dans l'hexane, filtré, séché. On recueille 21 g (85%) d'acide attendu, de point de fusion 213-214°C.

### c) 3-tert-butyl-4-benzyloxyacétophénone

De manière analogue à l'exemple 20(a), par réaction de 10 g (35 mmoles) de l'acide précédent, avec 5 ml (35 mmoles) de tétraméthylétain en présence de benzyl(chloro)bis (triphénylphosphine)palladium(II), on obtient 5,8 g (58%) de la cétone attendue sous forme d'une huile incolore.

### d) 3-tert-butyl-4-méthoxy(2'-bromo)acétophénone

De manière analogue à l'exemple 1(b), à partir de 5,8 g (20 mmoles) de la cétone obtenue en 21(c), on obtient 4,6 g (62%) du dérivé bromé attendu, sous forme d'une huile légèrement jaune.

### e) 2-hydroxy-4-[(3-tert-butyl-4-benzyloxybenzoyl)méthoxy]benzoate de benzyle

De manière analogue à l'exemple 19(a), par réaction de 4,6 g (13 mmoles) du dérivé bromé précédent, avec 3,1 g (13 mmoles) de 2,4-dihydroxybenzoate de benzyle, on obtient 5,4 g (81%) de l'ester attendu, de point de fusion 91-93°C.

### f) Acide 2-hydroxy-4-[2-hydroxy-2-(3-tert-butyl-4-hydroxyphényl)éthoxy]benzoïque

De manière analogue à l'exemple 1(d), par hydrogénation de 2 g (3,8 mmoles) de l'ester précédent, en présence de 1,5 g de palladium sur charbon à 10%, on obtient 1,9 g (90%) de l'acide attendu, de point de fusion 96-97°C.

### EXEMPLE 22

### Isomère (-) de l'acide 2-hydroxy-4-[2-hydroxy-2-(5,6,7,8-tétrahydro-5,5,8,8-tétraméthyl -2-naphtyl)éthoxy]benzoïque

### a) 2-(2-méthoxyéthoxyméthoxy)-4-(5,6,7,8-tétrahydro-5,5,8,8-tétraméthyl-2-naphtoylméthoxy)benzoate de benzyle

Dans un ballon, on introduit 1,65 g (55 mmoles) d'hydrure de sodium (80% dans l'huile) et 50 ml de DMF. On introduit goutte-à-goutte une solution de 23,6 g (50 mmoles) de 2-hydroxy-4-(5,6,7,8-tétrahydro-5,5,8,8-tétraméthyl-2-naphtoyl méthoxy)benzoate d'allyle dans 200 ml de DMF et agite jusqu'à cessation du dégagement gazeux. On ajoute ensuite goutte-à-goutte 6,3 ml (55 mmoles) de chlorure de 2-méthoxy éthoxyméthyle et agite 2 heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice, éluée avec un mélange dichlorométhane-éther éthylique (98/2). Après évaporation des solvants, on recueille 19,2 g (69%) de l'ester attendu, sous forme d'une huile légèrement jaune.

### b) 2-(2-méthoxyéthoxyméthoxy)-4-(2-hydroxy-2-(5,6,7,8-tétrahydro-5,5,8,8-tétraméthyl-2-naphtyl)éthoxy]benzoate de benzyle

De manière analogue à l'exemple 2(b), à partir de 10,3 g (18,3 mmoles) de l'ester précédent, on obtient 8,7 g (85%) du produit attendu, sous forme d'une huile jaune.

### c) 2-(2-méthoxyéthoxyméthoxy)-4-[2-(R)-a-méthoxyphénylacétyloxy-2-(5,6,7,8-tétrahydro-5,5,8,8-tétraméthyl-2-naphtyl)éthoxy]benzoate de benzyle

Dans un ballon, on introduit 10,5 g (18,6 mmoles) de 2-(2- méthoxyéthoxy méthoxy)-4-(2-hydroxy-2-(5,6,7,8-tétrahydro- 5,5,8,8-tétraméthyl-2-naphtyl)éthoxy]benzoate d'allyle, 3,1 g (18,6 mmoles) d'acide (R)-(-)a-méthoxyphénylacétique et 100 ml de dichlorométhane. On ajoute successivement 3,8 g (18,6 mmoles) de dicyclohexylcarbodiimide et 2,3 g (18,6 mmoles) de 4-diméthylaminopyridine et agite à température ambiante durant 4 heures. On verse le milieu réactionnel dans l'eau, extrait avec de l'éther éthylique, décante la phase organique, sèche sur sulfate de magnésium et évapore. Les deux diastéréoisomères formés sont séparés par chromatographie sur colonne de silice, en éluant avec un mélange hexane-éther éthylique (55/45). Après évaporation des solvants, on recueille :
- 5 g (38%) du diastéréoisomère (-) sous forme d'une huile légèrement jaune : α22D=-43°1(c=1,CH2Cl2)
- 4,8 g (36%) du diastéréoisomère (+) sous forme d'une huile légèrement jaune : α22D=+10°8(c=1,CH2Cl2)

### d) Diastéréoisomère (-) de 2-hydroxy-4-[2-(R)-a-méthoxyphénylacétyloxy-2-(5,6,7,8-tétrahydro-5,5,8,8-tétraméthyl-2-naphtyl)éthoxy]benzoate de benzyle

Dans un ballon, on introduit 2,5 g (3,5 mmoles) du diastéréoisomère (-) préparé à l'exemple 22(c) et 100 ml de dichlorométhane. On ajoute goutte-à-goutte 270 ml (3,5 mmoles) d'acide trifluoroacétique et agite 15 min. On verse dans l'eau, extrait avec de l'éther éthylique, décante la phase organique, sèche sur sulfate de magnésium, évapore. Le résidu est purifié par filtration sur silice, dans un mélange dichlorométhane-hexane (90/10). Après évaporation dessolvants, on recueille 2,1 g (97%) de l'ester attendu, sous forme d'une huile jaune:
α22D=-45°1(c=1,CH2Cl2)

### e) Isomère (-) de l'acide 2-hydroxy-4-[2-hydroxy-2-(5,6,7,8-tétrahydro-5,5,8,8-tétraméthyl -2-naphtyl)éthoxy]benzoïque

De manière analogue à l'exemple 3(c), à partir de 2 g (3,2 mmoles) de l'ester précédent, on obtient 1,1 g (92%) de l'acide (-) attendu, de point de fusion 199-200°C a20D=-7°6(c=1,DMF)

### EXEMPLE 23

### Isomère (+) de l'acide 2-hydroxy-4-[2-hydroxy-2-(5,6,7,8-tétrahydro-5,5,8,8-tétraméthyl -2-naphtyl)éthoxy]benzoïque

### a) Diastéréoisomère (+) de 2-hydroxy-4-[2-(R)-a-méthoxyphénylacétyloxy-2-(5,6,7,8-tetrahydro-5,5,8,8-tétraméthyl-2-naphtyl)éthoxy]benzoate de benzyle

De manière analogue à l'exemple 22(d), à partir de 4,4 g (6,3 mmoles) du diastéréoisomère (+) obtenu à l'exemple 22(c), on recueille 3,7 g (95%) de l'ester attendu, sous forme d'une huile jaune :
α22D=+19°5(c=1,CH2Cl2)

### b) Isomère (+) de l'acide 2-hydroxy-4-[2-hydroxy-2-(5,6,7,8-tétrahydro-5,5,8,8-tétraméthyl -2-naphtyl)éthoxy]benzoïque

De manière analogue à l'exemple 22(e), à partir de 3,5 g (5,6 mmoles) de l'ester précédent, on obtient 1,8 g (86%) de l'acide (+) attendu, de point de fusion 199-200°C :
α20D=+7°5(c=1,DMF)

### EXEMPLE 24

### Acide 2-hydroxy-4-[2-hydroxy-2(3-méthoxy-5,6,7,8-tétrahydro-5,5,8,8-tétraméthyl-2-naphtyl) éthoxy]benzoïque

### a) 3-méthoxy-5,6,7,8-tétrahydro-5,5,8,8-tétraméthyl-2-acétonaphtone

Dans un ballon, on introduit 990 mg (33 mmoles) d'hydrure de sodium (80% dans l'huile) et 20 ml de DMF. Sous courant d'azote, on ajoute goutte-à-goutte une solution de 6,8 g (27,6 mmoles) de 3-hydroxy-5,6,7,8-tétrahydro-5,5,8,8-tétraméthyl-2-acétonaphtone dans 75 ml de DMF et agite jusqu'à cessation du dégagement gazeux. On ajoute ensuite en refroidissant, 2,1 ml (33 mmoles) de lodométhane et agite à température ambiante 2 heures. On verse dans l'eau, extrait avec l'éther éthylique, décante la phase organique, sèche sur sulfate de magnésium, évapore. Le résidu obtenu est purifié par chromatographie sur colonne de silice, éluée avec un mélange dichlorométhane- hexane (40-60). Après évaporation des solvants, on recueille 6 g (84%) du produit attendu, de point de fusion 104-105°C.

### b) 2-(2'-bromoacéto)-3-méthoxy-5,6,7,8-tétrahydro-5,5,8,8-tétraméthylnaphtone

De manière analogue à l'exemple 1(b), à partir de 5,7 g (21,9 mmoles) de la cétone précédente, on obtient 7,4 g (100%) de dérivé bromé attendu, de point de fusion 99-100°C.

### c) 2-hydroxy-4-[3-méthoxy-5,6,7,8-tétrahydro-5,5,8,8-tétraméthyl-2-naphtoylméthoxy) benzoate de benzyle

De manière analogue à l'exemple 19(a), par réaction de 7,4 g (21,9 mmoles) du dérivé bromé précédent, avec 5,4 g (22 mmoles) de 2,4-dihydroxybenzoate de benzyle, on obtient 8,1 g (74%) de l'ester attendu, de point de fusion 118-119°C.

### d) Acide 2-hydroxy-4-(3-méthoxy-5,6,7,8-tétrahydro-5,5,8,8-tétraméthyl-2-naphtoylméthoxy)benzoïque

De manière analogue à l'exemple 17(c), à partir de 1 g (2 mmoles) de l'ester benzylique précédent, on obtient 640 mg (78%) de l'acide attendu, de point de fusion 200-201°C.

### e) Acide 2-hydroxy-4-[2-hydroxy-2(3-méthoxy-5,6,7,8-tétrahydro-5,5,8,8-tétraméthyl -2-naphtyl)éthoxy]benzoïque

De manière analogue à l'exemple 2(a), à partir de 1 g (2 mmoles) de l'acide précédent, on obtient 580 mg (70%) du produit attendu, de point de fusion 178-179°C.

### EXEMPLE 25

### Acide 2-méthoxy-4-[2-hydroxy-2-(5,6,7,8-tétrahydro-5,5,8,8-tétraméthyl-2-naphtyl) éthoxy]benzoïque

### a) 2-méthoxy-4-(5,6,7,8-tétrahydro-5,5,8,8-tétraméthyl-2- naphtylméthoxy)benzoate de benzyle

De manière analogue à l'exemple 1(a), par réaction de 1,9 g (4 mmoles) de 2-hydroxy-4-(5,6,7,8-tétrahydro-5,5,8,8-tétraméthyl-2-naphtoylméthoxy)benzoate de benzyle, avec 280 ml (4,4 mmoles) de iodométhane, on obtient 1,8 g (93%) du produit attendu, de point de fusion 112-113°C.

### b) Acide 2-méthoxy-4-[2-hydroxy-2-(5,6,7,8-tétrahydro-5,5,8,8-tétraméthyl-2-naphtyl) éthoxy]benzoïque

De manière analogue à l'exemple 1(d), à partir de 1,7 g (3,5 mmoles) de l'ester benzylique précédent, on obtient 1,1 g (79%) de l'acide attendu, de point de fusion 150-151°C.

### EXEMPLE 26

### Acide 2-hydroxy-4-[2-hydroxy-2-(3-hydroxy-5,6,7,8-tétrahydro-5,5,8,8-tétraméthyl -2-naphtyl)éthoxy]benzoïque

### a) 2-hydroxy-4-[3-hydroxy-5,6,7,8-tétrahydro-5,5,8,8-tétraméthyl-2-naphtoylméthoxy) benzoate de benzyle

Dans un ballon, on introduit 4 g (8 mmoles) de 2-hydroxy-4-(3-méthoxy-5,6,7,8-tétrahydro-5,5,8,8-tétraméthyl-2-naphtoylméthoxy)benzoate de benzyle et 20 ml de dichlorométhane. A -78°C et sous courant d'azote, on ajoute goutte-à-goutte 24 ml (24 mmoles) d'une solution de trichlorure de bore dans le THF (1M) et laisse remonter la température à -20°C, puis verse le milieu réactionnel dans l'eau glacée. On extrait à l'éther éthylique, décante la phase organique, sèche sur sulfate de magnésium, évapore. Le résidu obtenu est purifié par chromatographie sur colonne de silice, éluée avec un mélange dichlorométhane-hexane (50/50). On obtient 3,1 g (80%) de l'ester attendu, de point de fusion 127-128°C.

### b) Acide 2-hydroxy-4-[3-hydroxy-5,6,7,8-tétrahydro-5,5,8,8-tétraméthyl-2-naphtoylméthoxy)benzoïque

De manière analogue à l'exemple 17(c), à partir de 2,8 g (5,7 mmoles) de l'ester benzylique précédent, on obtient 2 g (88%) de l'acide attendu qui fond à 194-195°C.

### c) Acide 2-hydroxy-4-[2-hydroxy-2-(3-hydroxy-5,6,7,8-tétrahydro-5,5,8,8-tétraméthyl-2-naphtyl)éthoxy]benzoïque

De manière analogue à l'exemple 2(b), à partir de 1 g (2,5 mmoles) de l'acide obtenu en 26(b), on obtient 270 mg (27%) d'acide attendu, de point de fusion 110-111°C.

### EXEMPLE 27

### Acide 2-hydroxy-4-[2-amino-2-(5,6,7,8-tétrahydro-5,5,8,8-tétraméthyl-2-naphtyl) éthoxy]benzoïque

### a) 2-hydroxy-4-[2-hydroxy-2-(5,6,7,8-tétrahydro-5,5,8,8-tétraméthyl-2-naphtyl)éthoxy] benzoate de benzyle

De manière analogue à l'exemple 2(b), à partir de 9,44 g (20 mmoles) de 2-hydroxy-4-(5,6,7,8-tétrahydro-5,5,8,8-tétraméthyl-2-naphtoylméthoxy)benzoate de benzyle, on obtient 9,4 g (100%) de l'ester attendu, sous forme d'une huile légèrement jaune.

### b) 2-hydroxy-4-[2-méthanesulfonyloxy-2-(5,6,7,8-tétrahydro-5,5,8,8-tétraméthyl-2-naphtyl) éthoxy]benzoate de benzyle

Dans un ballon, on introduit 1,8 g (3,8 mmoles) de l'ester précédent, 920 ml (11,4 mmoles) de pyridine et 100 ml de dichlorométhane. A 0°C, on ajoute goutte-à-goutte une solution de 350 ml (4,6 mmoles) de chlorure de méthane sulfonyl dans 50 ml de dichlorométhane et agite à température ambiante 4 heures. On évapore à sec, reprend par éther éthylique, lave la phase organique à l'eau, sèche sur sulfate de magnésium, évapore. On recueille 2,1 g (100%) du produit attendu, sous forme d'une huile.

### c) 2-hydroxy-4-[2-azido-2-(5,6,7,8-tétrahydro-5,5,8,8-tétraméthyl-2-naphtyl)éthoxy]benzoate de benzyle

Dans un ballon, on introduit 2,1 g (3,8 mmoles) de l'ester obtenu en 27(b), 50 ml de DMF et 750 mg (11,4 mmoles) d'azoture de sodium. On agite à température ambiante 12 heures, verse le milieu réactionnel dans l'eau, extrait avec de l'éther éthylique, décante la phase organique, sèche sur sulfate de magnésium, évapore. Le résidu obtenu est purifié par chromatographie sur colonne de silice, éluée avec un mélange dichlorométhane-hexane (40/60). Après évaporation des solvants, on recueille 1,2 g (67%) du produit attendu, sous forme d'une huile incolore.

### d) 2-hydroxy-4-[2-amino-2-(5,6,7,8-tétrahydro-5,5,8,8-tétraméthyl-2-naphtyl)éthoxy] benzoate de benzyle

Dans un ballon, on introduit 2,2 g (4,4 mmoles) de l'ester précédent, 1,2 g (4,4 mmoles) de triphénylphosphine, 120 ml (6,6 mmoles) d'eau et 100 ml de THF. On agite à température ambiante 24 heures, évapore à sec et chromatographie sur colonne de silice en éluant avec un mélange hexane-éther éthylique (40/60). Après évaporation des solvants, on recueille 1 g (48%) du produit attendu, sous forme d'une huile légèrement jaune.

### e) Acide 2-hydroxy-4-[2-amino-2-(5,6,7,8-tétrahydro-5,5,8,8-tétraméthyl-2-naphtyl) éthoxy]benzoïque

Dans un ballon, on introduit 800 mg (1,7 mmole) de l'ester précédent et 30 ml d'une solution de soude méthanolique 2N. On chauffe à reflux durant 4 heures, évapore le milieu réactionnel, reprend par eau, neutralise à pH=5 avec acide chlorhydrique 1N, extrait avec de l'éther éthylique. On décante la phase organique, lave à l'eau, sèche sur sulfate de magnésium, évapore. On triture le résidu dans le minimum d'éther éthylique, filtre, sèche. On recueille 110 mg (17%) du produit attendu, de point de fusion 241-242°C.

### EXEMPLE 28

### Acide 2-hydroxy-4-[2-hydroxy-2-(5,6,7,8-tétrahydro-5,5,8,8-tétraméthyl-2-naphtyl) propyloxy]benzoïque

Dans un tricol, sous courant d'azote, on introduit 1 g (2,6 mmoles) d'acide 2-hydroxy-4-(5,6,7,8-tétrahydro-5,5,8,8-tétraméthyl-2-naphtoylméthoxy)benzoïque et 50 ml de THF. A - 78°C, on ajoute goutte-à-goutte 5,3 ml (8,3 mmoles) d'une solution de méthyllithium dans le THF (1,6M) et agite 12 heures à température ambiante. On verse le milieu réactionnel dans l'eau glacée, acidifiée à pH=1, extrait avec de l'éther éthylique, décante la phase organique, sèche sur sulfate de magnésium et évapore. Le résidu obtenu est recristallisé dans le cyclohexane, on recueille 900 mg (86%) du produit attendu, de point de fusion 170-171°C.

### EXEMPLE 29

### Acide 2-hydroxy-4-[2-hydroxy-2-(5,6,7,8-tétrahydro-5,5,8,8-tétraméthyl-2-naphtyl) hexyloxy]benzoïque

De manière analogue à l'exemple 28, par réaction de 1,1 g (2,9 mmoles) d'acide 2-hydroxy-4-(5,6,7,8,-tétrahydro-5,5,8,8-tétraméthyl-2-naphtylméthoxy)benzoïque, avec 5,4 ml (8,6 mmoles) d'une solution de n-butyllithium (1,6M) dans l'hexane, on obtient 140 mg (11%) d'acide attendu, de point de fusion 142-143°C.

### EXEMPLE 30

### Acide 2-hydroxy-4-[2-hydroxy-2-(5,6,7,8-tétrahydro-5,5,8,8-tétraméthyl-2-naphtyl) éthylamino]benzoïque

Dans un ballon, on introduit 2,05 g (5 mmoles) de 2-hydroxy-4-(5,6,7,8-tétrahydro-5,5,8,8-tétraméthyl-2-naphtoylcarboxamido)benzoate de méthyle et 50 ml de dioxane. On ajoute par petites quantités, 1,9 g (50 mmoles) de borohydrure de sodium et agite 30 min à température ambiante. On refroidit à 0°C et ajoute goutte-à-goutte 2,9 ml (50 mmoles) d'acide acétique et agite 4 heures à température ambiante. On verse dans l'eau glacée, extrait avec de l'éther éthylique, décante la phase organique, lave à l'eau, sèche sur sulfate de magnésium, évapore. Le résidu obtenu est trituré dans un mélange hexane-éther éthylique (50-50), filtré, sèche. On recueille 1,7 g (89%) d'acide 2-hydroxy-4-[2-hydroxy-2-(5,6,7,8-tétrahydro-5,5,8,8-tétraméthyl-2- naphtyl)éthylamino]benzoïque, de point de fusion 165-166°C.

### EXEMPLE 31

### Acide 2-hydroxy-4-[[2-hydroxy-2-[3-(1-adamantyl)-4-méthoxyphényl]éthoxy]]benzoïque

Dans un réacteur, on introduit 1,3 g (2,98 mmoles) d'acide 2-hydroxy-4-[[3-(1-adamantyl)-4-méthoxybenzoyl]méthoxy]benzoïque, 200 mg de palladium sur charbon à 10% et 50 ml de dioxanne. On hydrogène à température ambiante et sous une pression de 7 bars pendant 4 heures, filtre le catalyseur, lave avec 50 ml de THF, évapore les filtrats. Le résidu obtenu est purifié par chromatographie sur colonne de silice, en éluant avec un mélange dichlorométhane-méthanol (98/2). Après évaporation des solvants, on recueille 1 g (77%) de l'acide attendu, de point de fusion 178-179°C.

### EXEMPLE 32

### Acide 2-hydroxy-4-[[2-[3-(1-adamantyl)-4-méthoxyphenyl]éthoxy]]benzoïque

Dans un réacteur, on introduit 1,3 g (2,98 mmoles) d'acide 2-hydroxy-4-[[3-(1-adamantyl)-4-méthoxybenzoyle]méthoxy]benzoïque, 800 mg de palladiumm sur charbon à 10% et 100 ml de dioxanne. On hydrogène à température ambiante, sous une pression de 7 bars pendant 4 heures, filtre le catalyseur, lave avec 50 ml de THF, évapore les filtrats. Le résidu obtenu est purifié par chromatographie sur colonne de silice, en éluant avec un mélange dichlorométhane-méthanol (98/2). Après évaporation des solvants, on recueille 790 mg (60%) de l'acide attendu, de point de fusion 210-211°C.

### B. EXEMPLES DE FORMULATION

### 1) VOIE ORALE

### (a) Comprimé de 0,8 g

| | |
|---|---|
| Composé de l'exemple 1 | 0,500 g |
| Amidon prégélatinisé | 0,100 g |
| Cellulose microcristalline | 0,115 g |
| Lactose | 0,075 g |
| Stéarate de magnésium | 0,010 g |

Dans cet exemple, le composé de l'exemple 1 peut être remplacé par la même quantité du composé de l'exemple 23.

### (b) Suspension buvable en ampoules de 5 ml

| | |
|---|---|
| Composé de l'exemple 2 | 0,500 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p | 5 ml |

Dans cet exemple, le composé de l'exemple 2 peut être remplacé par la même quantité du composé de l'exemple 28.

### (c) Comprimé de 0,2 g

| | |
|---|---|
| Composé de l'exemple 3 | 0,001 g |
| Amidon | 0,114 g |
| Phosphate bicalcique | 0,020 g |
| Silice | 0,020 g |
| Lactose | 0,030 g |
| Talc | 0,010 g |
| Stéarate de magnésium | 0,005 g |

Dans cet exemple, le composé de l'exemple 3 peut être remplacé par la même quantité du composé de l'exemple 15.

### (d) Suspension buvable en ampoules de 10 ml

| | |
|---|---|
| Composé de l'exemple 4 | 0,200 g |
| Glycérine | 1,000 g |
| Sorbitol à 70 % | 1,000 g |
| Saccharinate de sodium | 0,010 g |
| Parahydroxybenzoate de méthyle | 0,080 g |
| Arôme qs | |
| Eau purifiée qsp | 10 ml |

### (e) Comprimé non soluble de 0,5 g

| | |
|---|---|
| Composé de l'exemple 5 | 0,050 g |
| Lactose | 0,082 g |
| Acide stéarique | 0,003 g |
| Talc purifié | 0,015 g |
| Edulcorant qs | |
| Colorant qs | |
| Amidon de riz qsp | 0,500 g |

### (f) Comprimé non soluble de 0,8 g

| | |
|---|---|
| Composé de l'exemple 6 | 0,010 g |
| Lactose qsp | 0,800 g |
| Gomme arabique à 20 % dans l'eau | 0,080 g |
| Paraffine liquide | 0,004 g |
| Talc purifié | 0,016 g |
| Amidon qsp | 0,800 g |

### (g) Capsules de 1 g contenant 0,5 g

| Contenu de la capsule : Suspension huileuse | |
|---|---|
| Composé de l'exemple 7 | 0,005 g |
| Huile de paraffine qsp | 0,500 g |

L'enveloppe de la capsule est fabriquée par moulage puis séchage d'un mélange approprié composé de : gélatine, glycérine, eau et conservateur.

### (h) Gélule contenant 0,3 g de poudre

| Composition de la poudre : | |
|---|---|
| Composé de l'exemple 14 | 0,100 g |
| Amidon du maïs | 0,060 g |
| Lactose qsp | 0,300 g |

La poudre est conditionnée dans une gélule composée de gélatine, de TiO₂ et d'un conservateur.

### (i) Gélule de 0,30 ml

### Enveloppe standard calibre M°3 opaque

| Contenu poudre à 0,1% en poids d'actif : | |
|---|---|
| Composé de l'exemple 17 | 0,3 mg |
| Stéarate de magnésium | 30 mg |
| Silice vendue par la Société DEGUSSA sous la dénomination Aerosil 200 | 30,0 mg |
| Lactose qsp | 0,3 ml |

### 2 - VOIE TOPIQUE

### (a) Crème Eau-dans-l'Huile non ionique

| | |
|---|---|
| Composé de l'exemple 1 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p. | 100 g |

Dans cet example, le composé de l'exemple 1 peut être remplacé par la même quantité du composé de l'exemple 8.

### (b) Crème Huile dans-l'Eau non ionique

| | |
|---|---|
| Composé de l'exemple 2 | 1,000 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p. | 100 g |

Dans cet exemple, le composé de l'exemple 2 peut être remplacé par la même quantité du composé de l'exemple 10.

### (c) Lotion

| | |
|---|---|
| Composé de l'exemple 19 | 0,100 g |
| Polyéthylène glycol (PEG 400) | 69,900 g |
| Ethanol 95 % | 30,000 g |

Dans cet exemple, le composé de l'exemple 19 peut être remplacé par la même quantité du composé de l'exemple 21.

### (d) Onguent

| | |
|---|---|
| Composé de l'exemple 20 | 0,020 g |
| Myristate d'isopropyle | 81,700 g |
| Huile de vaseline fluide | 9,100 g |
| Silice vendue par la Société DEGUSSA sous la dénomination "Aérosil 200" | 9,180 g |

Dans cet exemple, le composé de l'exemple 20 peut être remplacé par la même quantité du composé de l'exemple 11.

### (e) Onguent

| | |
|---|---|
| Composé de l'exemple 24 | 0,300 g |
| Vaseline blanche codex qsp | 100 g |

Dans cet exemple le composé de l'exemple 24 peut être remplacé par la même quantité du composé de l'exemple 31.

### (f) Onguent hydrophobe

| | |
|---|---|
| Composé de l'exemple 25 | 0,300 g |
| Myristate d'isopropyle | 36,400 g |
| Huile de silicone vendue par la Société RHONE POULENC sous la dénomination "Rhodorsil 47 V 300" | 36,400 g |
| Cire d'abeille | 13,600 g |
| Huile de silicone vendue par la Société GOLDSCHMIDT sous la dénomination "Abil 300.000 cst" qsp | 100 g |

Dans cet exemple, le composé de l'exemple 25 peut être remplacé par la même quantité du composé de l'exemple 32.

### (g) Onguent hydrophile

| | |
|---|---|
| Composé de l'exemple 27 | 0,005 g |
| Eucerine anhydre | 60,000 g |
| Cire microcristalline | 15,000 g |
| Huile de vaseline qsp | 100,000 g |

### (h) Onguent

| | |
|---|---|
| Composé de l'exemple 22 | 0,050 g |
| Alcool stéarylique | 3,000 g |
| Lanoline | 5,000 g |
| Vaseline | 15,000 g |
| Eau distillée qsp | 100,000 g |

### (i) Onguent hydrophobe

| | |
|---|---|
| Composé de l'exemple 26 | 1,000 g |
| Huile de vaseline fluide | 9,100 g |
| Silice vendue par la Société DEGUSSA sous la dénomination Aérosil 200 | 9,180 g |
| Myristate d'isopropyle qsp | 100,000 g |

### (j) Crème H/E Anionique

| | |
|---|---|
| Composé de l'exemple 30 | 0,050 g |
| Dodecyl sulfate de sodium | 0,800 g |
| Glycérol | 2,000 g |
| Alcool stéarylique | 20,000 g |
| Triglycérides d'acides caprique/caprilique vendus par la Société DYNAMIT NOBEL sous le nom Miglyol 812 | 20,000 g |
| Conservateurs qs | |
| Eau déminéralisée qsp | 100,000 g |

### (k) Onguent hydroéliminable

| | |
|---|---|
| Composé de l'exemple 29 | 0,500 g |
| PEG 400 | 59,500 g |
| PEG 4000 | 25,000 g |
| Huile de vaseline | 15,000 g |

## Revendications (Revendications pour l'(les) Etat(s) contractant(s) suivant(s): AT, BE, CH, LI, DE, DK, FR, GB, GR, IT, NL, PT, SE)

1. Composés bi-aromatiques, caractérisés par le fait qu'ils répondent à la formule générale suivante : 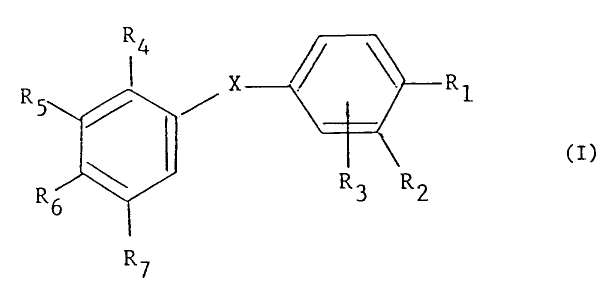 dans laquelle :
R₁ représente le radical - CH₃, le radical - CH₂OH, le radical - COR₈, ou le radical - CH₂OCOR₉,
R₈ représentant un atome d'hydrogène, OH, - OR₁₀, 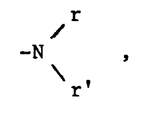 ou un radical alkyle inférieur,
R₁₀ représentant un radical alkyle ayant de 1 à 20 atomes de carbone, un radical alkényle ayant de 2 à 20 atomes de carbone, un radical aryle ou aralkyle,
r et r', identiques ou différents, représentant un atome d'hydrogène, un radical alkyle inférieur, un radical aryle, un radical aralkyle, un reste d'α-aminoacide, un reste de sucre ou un hétérocycle ou r et r' pris ensemble formant un hétérocycle,
R₉ représentant un radical alkyle ayant de 1 à 20 atomes de carbone, un radical alkényle ayant de 2 à 20 atomes de carbone ou un reste de sucre,
R₂ et R₃ représentent - OR₁₁ ou - OCOR₁₁
R₁₁ représentant un atome d'hydrogène, un radical alkyle inférieur, un radical fluoroalkyle ayant de 1 à 6 atomes de carbone et de 3 à 7 atomes de fluor, un radical aryle ou un radical aralkyle,
R₃ pouvant représenter en outre un atome d'hydrogène,
R₄ représente un atome d'hydrogène, OH, un radical alkyle inférieur, un radical alkoxy ayant de 1 à 6 atomes de carbone, un atome de fluor, de chlore ou le groupe -CF₃,
R₅ et R₇ représentent un atome d'hydrogène, OH, un radical alkoxy ayant de 1 à 6 atomes de carbone, un radical alkyle α-substitué ayant de 3 à 12 atomes de carbone ou un radical alkyle α-α'disubstitué ayant de 4 à 12 atomes de carbone, un radical cycloalkyle ayant de 3 à 12 atomes de carbone, un radical mono ou polycyclique ayant de 5 à 12 atomes de carbone lié au noyau phényle par un carbone tertiaire, R₅ et R₇ ne pouvant représenter simultanément OH ou alcoxy,
R₆ représente un atome d'hydrogène, OH, un radical alkyle inférieur, un radical alkoxy ayant de 1 à 6 atomes de carbone, un radical cycloalkyle ayant de 3 à 12 atomes de carbone, un radical monohydroxyalkyle, un radical polyhydroxyalkyle, un atome de fluor, un atome de chlore, un radical alkényle ayant de 2 à 6 atomes de carbone ou un radical alkényloxy ayant de 2 à 6 atomes de carbone, R₄, R₅, R₆ et R₇ ne pouvant représenter simultanément un atome d'hydrogène,
R₅ et R₆ ou R₆ et R₇ pris ensemble peuvent former avec le cycle aromatique adjacent un cycle à 5 ou 6 chainons éventuellement substitué par des groupes méthyle et/ou éventuellement interrompu par un atome d'oxygène ou de soufre,
X est un radical divalent qui peut être lu de gauche à droite ou inversement choisi dans le groupe constitué par :
(i) -C(R₁₃R₁₄)-C(R₁₆R₁₈)-W-
(ii) -C(R₁₄R₁₆)-W-C(R₁₈R₁₉)-
(iii) -C(R₁₃R₁₄)-C(R₁₅R₁₆)-C(R₁₈R₂₀)-
(iv) -CR₁₇=CR₂₁-C(R₁₃R₁₄)-
dans lesquels :
W représente un atome d'oxygène, le groupe -NR₁₂ ou le groupe S(O)n, n étant 0, 1 ou 2,
R₁₃, R₁₅ et R₂₀ représentent un atome d'hydrogène, le radical -OR₁₁, -OCOR₁₁, -NHCOR₁₁, un radical 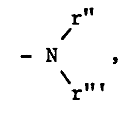 un radical aralkyle, un radical alkyle inférieur, un radical monohydroxyalkyle ou un radical polyhydroxyalkyle,
r'' et r''' identiques ou différents, représentant un atome d'hydrogène, un radical alkyle inférieur, un radical alkényle ayant de 2 à 6 atomes de carbone ou un radical alkynyle ayant de 2 à 6 atomes de carbone,
R₁₄, R₁₆, R₁₈ et R₁₉ représentent un atome d'hydrogène, un radical aralkyle, un radical alkyle inférieur, un radical monohydroxyalkyle ou polyhydroxyalkyle,
lorsque X représente (i), R₁₃ et R₁₄ peuvent former un groupe =N-OR₁₁ ou un groupe =N-OCOR₁₁,
lorsque X représente (iii) ou (iv), R₁₄, R₁₆ et R₁₈ peuvent également représenter le radical -OR₁₁ ou le radical -OCOR₁₁, ou encore R₁₃, R₁₄ ou R₁₅, R₁₆ pris ensemble peuvent former un groupe =NOR₁₁ ou un groupe =N-OCOR₁₁,
R₁₂ représentant un atome d'hydrogène, un radical alkyle inférieur, un radical aralkyle, un radical alkényle ayant de 2 à 6 atomes de carbone, un radical alkynyle ayant de 2 à 6 atomes de carbone ou un radical fluoroalkyle ayant de 1 à 6 atomes de carbone et de 3 à 7 atomes de fluor,
R₁₇ représentant un atome d'hydrogène, un groupe hydroxyle, un radical alkyle inférieur ou un radical alkoxy ayant de 1 à 6 atomes de carbone,
R₂₁ représentant un atome d'hydrogène ou un radical alkyle inférieur,
et les sels des composés de formule (I) lorsque R₁ représente une fonction acide carboxylique ou lorsque R₁₃, R₁₅ ou R₂₀ représente une fonction amine et les isomères optiques des composés de formule (I).

2. Composés selon la revendication 1, caractérisés par le fait qu'ils se présentent sous forme de sels d'un métal alcalin ou alcalino-terreux ou encore de zinc ou d'une amine organique.

3. composés selon la revendication 1, caractérisés par le fait qu'ils se présentent sous forme de sels d'un acide minéral ou organique choisi parmi l'acide chlorhydrique, sulfurique, acétique, citrique, fumarique, hémisuccinique, maléique et mandélique.

4. Composés selon la revendication 1, caractérisés par le fait que le radical alkyle inférieur a de 1 à 6 atomes de carbone et est pris dans le groupe constitué par les radicaux méthyle, éthyle, isopropyle, butyle et tertiobutyle.

5. Composés selon la revendication 1, caractérisés par le fait que le radical alkoxy ayant de 1 à 6 atomes de carbones est pris dans le groupe constitué par un radical méthoxy, éthoxy, isopropoxy et butoxy.

6. Composés selon la revendication 1, caractérisés par le fait que le radical alkyle α,α'-disubstitué est pris dans le groupe constitué par : un radical tert-butyle, 1,1-diméthyl propyle, 1-méthyl 1-éthyl propyle, 1-méthyl 1-éthyl hexyle et 1,1-diméthyl décyle.

7. Composés selon la revendication 1, caractérisés par le fait que le radical cycloalkyle mono ou polycyclique ayant de 5 à 12 atomes de carbone dont le carbone de liaison est trisubstitué, est le radical 1-méthyl cyclohexyle ou 1-adamantyle.

8. Composés selon la revendication 1, caractérisés par le fait que le radical monohydroxyalkyle est le radical 2-hydroxyéthyle, 2-hydroxypropyle ou 3-hydroxypropyle.

9. Composés selon la revendication 1, caractérisés par le fait que le radical polyhydroxyalkyle comporte de 2 à 6 atomes de carbone et de 2 à 5 groupes hydroxyles et est pris dans le groupe constitué par le radical 2,3-dihydroxypropyle, 2,3,4-trihydroxybutyle, 2,3,4,5-tétrahydroxypentyle et le reste du pentaérythritol.

10. Composés selon la revendication 1, caractérisés par le fait que le radical aryle est un radical phényle éventuellement substitué par au moins un atome d'halogène, un hydroxyle ou une fonction nitro.

11. Composés selon la revendication 1, caractérisés par le fait que le radical aralkyle est le radical benzyle ou phénéthyle éventuellement substitué par au moins un atome d'halogène, un hydroxyle ou une fonction nitro.

12. Composés selon la revendication 1, caractérisés par le fait que le radical alkynyle ayant de 2 à 6 atomes de carbone est le radical propargyle.

13. Composés selon la revendication 1, caractérisés par le fait que le radical alkényle ayant de 2 à 6 atomes de carbone est pris dans le groupe constitué par le radical vinyle, allyle et 2-butényle.

14. Composés selon la revendication 1, caractérisés par le fait que le radical fluoroalkyle inférieur est un radical ayant de 1 à 6 atomes de carbone et de 3 à 7 atomes de fluor.

15. Composés selon la revendication 1, caractérisés par le fait que l'hétérocycle est pris dans le groupe constitué par un radical pipéridino, morpholino, pyrrolidino ou pipérazino, éventuellement substitué en position 4 par un radical alkyle en C₁-C₆ ou mono ou polyhydroxyalkyle.

16. Composés selon l'une quelconque des revendications précédentes, caractérisés par le fait qu'ils sont pris dans le groupe constitué par :
Acide 2-hydroxy-4-[2-hydroxy-2-(5,6,7,8-tétrahydro-5,5,8,8-tétraméthyl-2-naphtyl)éthoxy]benzoïque ;
2-hydroxy-4-[2-hydroxy-2-(5,6,7,8-tétrahydro-5,5,8,8-tétraméthyl-2-naphtyl)éthoxy]benzoate de méthyle ;
Acide 2-hydroxy-4-[2-hydroxyimino-2-(5,6,7,8-tétrahydro-5,5,8,8-tétraméthyl-2-naphtyl)éthoxy]benzoïque ;
Acide 2-acétyloxy-4-[2-acétyloxy-2-(5,6,7,8-tétrahydro-5,5,8,8-tétraméthyl-2-naphtyl)éthoxy]benzoïque ;
Acide 2-hydroxy-4-[2-acétyloxy-2-(5,6,7,8-tétrahydro-5,5,8,8-tétraméthyl-2-naphtyl)éthoxy]benzoïque ;
Acide 2-acétyloxy-4-[2-hydroxy-2-(5,6,7,8-tétrahydro-5,5,8,8-tétraméthyl-2-napthyl)éthoxy]benzoïque.
Acide-2-hydroxy-4-[2-(5,6,7,8-tétrahydro-5,5,8,8-tétraméthyl -2-naphtyl)éthoxy]benzoïque ;
2-hydroxy-4-[2-hydroxy-2-(5,6,7,8-tétrahydro-5,5,8,8-tétraméthyl-2-naphtyl)éthoxy]benzyl alcool ;
Acétate de 2-acétyloxy-4- [2-hydroxy-2-(5,6,7,8-tétrahydro-5,5,8,8-tétraméthyl-2-naphtyl)éthoxy]benzyl alcool ;
Piperidinylamide de l'acide 2-hydroxy-4- [2-hydroxy-2-(5,6,7,8-tétrahydro-5,5,8,8-tétraméthyl-2-naphtyl)éthoxy]benzoïque ;
Morpholinylamide de l'acide 2-hydroxy-4-[2-hydroxy-2-(5,6,7,8-tétrahydro-5,5,8,8-tétraméthyl-2-naphtyl)éthoxy]benzoïque .
2-hydroxy-4-[2-hydroxy-2-(5,6,7,8-tétrahydro-5,5,8,8-tétraméthyl-2-naphtyl)éthoxy]benzamide ;
N-éthyl amide de l'acide 2-hydroxy-4- [2-hydroxy-2-(5,6,7,8-tétrahydro-5,5,8,8-tétraméthyl-2-naphtyl)éthoxy]benzoïque ;
2-hydroxy-4-[2-hydroxy-2-(4,4-diméthylthiochroman-6-yl) éthoxy]benzoate de méthyle ;
Acide 2-hydroxy-4-[2-hydroxy-2-(4,4-diméthylthiochroman-6-yl) éthoxy]benzoïque ;
Acide 2-hydroxy-4-[3-hydroxy-3-(5,6,7,8-tétrahydro-5,5,8,8-tétraméthyl-2-naphtyl)propyl]benzoïque ;
Acide 2-hydroxy-4-[2-hydroxy-2-(3,5-di-tert-butyl-4-hydroxyphényl)éthoxy]benzoïque ;
2-hydroxy-4-[2-hydroxy-2-(5,6,7,8-tétrahydro-5,5,8,8-tétraméthyl-2-naphtyl)éthoxy]toluène ;
2,6-dihydroxy-4-[2-hydroxy-2-(5,6,7,8-tétrahydro-5,5,8,8-tétraméthyl-2-naphtyl)éthoxy]benzoate de méthyle
Acide 2-hydroxy-4-[2-hydroxy-2-(3-tert-butyl-4-méthoxyphényl) éthoxy]benzoïque ;
Acide 2-hydroxy-4-[2-hydroxy-2-(3-tert-butyl-4-hydroxyphényl) éthoxy]benzoïque ;
Isomère (-) de l'acide 2-hydroxy-4-[2-hydroxy-2-(5,6,7,8-tétrahydro-5,5,8,8-tétraméthyl-2-naphtyl)éthoxy]benzoïque ;
Isomère (+) de l'acide 2-hydroxy-4-[2-hydroxy-2-(5,6,7,8-tétrahydro-5,5,8,8-tétraméthyl-2-naphtyl)éthoxy]benzoïque ;
Acide 2-hydroxy-4-[2-hydroxy-2(3-méthoxy-5,6,7,8-tétrahydro-5,5,8,8-tétraméthyl-2-naphtyl)éthoxy]benzoïque ;
Acide 2-méthoxy-4-[2-hydroxy-2-(5,6,7,8-tétrahydro-5,5,8,8-tétraméthyl-2-naphtyl)éthoxy]benzoïque ;
Acide 2-hydroxy-4-[2-hydroxy-2-(3-hydroxy-5,6,7,8-tétrahydro-5,5,8,8-tétraméthyl-2-naphtyl)éthoxy]benzoïque ;
Acide 2-hydroxy-4- [2-amino-2-(5,6,7,8-tétrahydro-5,5,8,8-tétraméthyl-2-naphtyl)éthoxy]benzoïque ;
Acide 2-hydroxy-4-[2-hydroxy-2-(5,6,7,8-tétrahydro-5,5,8,8-tetraméthyl-2-naphtyl)propyloxy]benzoïque ;
Acide 2-hydroxy-4-[2-hydroxy-2-(5,6,7,8-tétrahydro-5,5,8,8-tétraméthyl-2-naphtyl)hexyloxy]benzoïque ;
Acide 2-hydroxy-4-[2-hydroxy-2-(5,6,7,8-tétrahydro-5,5,8,8-tétraméthyl-2-naphtyl)éthylamino]benzoïque ;
Acide 2-hydroxy-4-[[2-hydroxy-2-[3-(1-adamantyl)-4- méthoxyphényl] éthoxy]]benzoïque ;
Acide 2-hydroxy-4-[[2-[3-(1-adamantyl)-4-méthoxyphenyl] éthoxy]]benzoïque.

17. Composition pharmaceutique, caractérisée par le fait qu'elle contient dans un véhicule approprié, pour une administration par voie entérale, parentérale, topique ou oculaire, au moins un composé de formule (I) selon l'une quelconque des revendications 1 à 16.

18. Composition selon la revendication 17, caractérisée par le fait qu'elle contient de 0,001 à environ 5% en poids d'un composé de formule (I).

19. Utilisation d'un composé selon l'une quelconque des revendications 1 à 16 pour la préparation d'une composition pharmaceutique destinée au traitement des affections dermatologiques, rhumatismales, respiratoires ainsi qu'ophtalmologiques.

20. Composition cosmétique pour l'hygiène corporelle et capillaire, caractérisée par le fait qu'elle contient, dans un véhicule cosmétique approprié, au moins un composé de formule (I) selon l'une quelconque des revendications 1 à 16.

21. Composition cosmétique selon la revendication 20, caractérisée par le fait qu'elle contient le composé de formule (I) à une concentration comprise entre 0,001 et 3 % en poids.

## Revendications (Revendications pour l'(les) Etat(s) contractant(s) suivant(s): ES)

1. Composition cosmétique pour l'hygiène corporelle et capillaire, caractérisée par le fait qu'elle contient, dans un véhicule cosmétique approprié, au moins un composé de formule (I) : 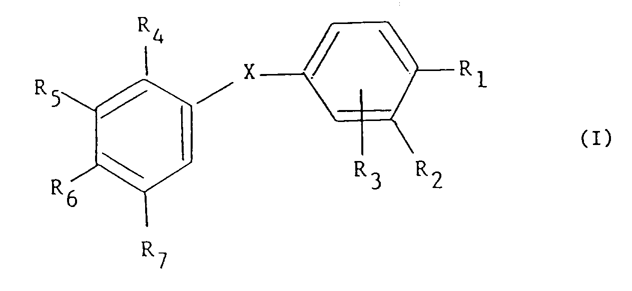 dans laquelle :
R₁ représente le radical -CH₃, le radical -CH₂OH, le radical -COR₈, ou le radical -CH₂OCOR₉,
R₈ représentant un atome d'hydrogène, OH, -OR₁₀, 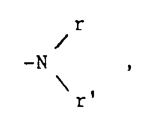 ou un radical alkyle inférieur,
R₁₀ représentant un radical alkyle ayant de 1 à 20 atomes de carbone, un radical alkényle ayant de 2 à 20 atomes de carbone, un radical aryle ou aralkyle,
r et r', identiques ou différents, représentant un atome d'hydrogène, un radical alkyle inférieur, un radical aryle, un radical aralkyle, un reste d'α-aminoacide, un reste de sucre ou un hétérocycle ou r et r' pris ensemble formant un hétérocycle,
R₉ représentant un radical alkyle ayant de 1 à 20 atomes de carbone, un radical alkényle ayant de 2 à 20 atomes de carbone ou un reste de sucre,
R₂ et R₃ représentent -OR₁₁ ou -OCOR₁₁
R₁₁ représentant un atome d'hydrogène, un radical alkyle inférieur, un radical fluoroalkyle ayant de 1 à 6 atomes de carbone et de 3 à 7 atomes de fluor, un radical aryle ou un radical aralkyle,
R₃ pouvant représenter en outre un atome d'hydrogène,
R₄ représente un atome d'hydrogène, OH, un radical alkyle inférieur, un radical alkoxy ayant de 1 à 6 atomes de carbone, un atome de fluor, de chlore ou le groupe -CF₃,
R₅ et R₇ représentent un atome d'hydrogène, OH, un radical alkoxy ayant de 1 à 6 atomes de carbone, un radical alkyle α-substitué ayant de 3 à 12 atomes de carbone ou un radical alkyle α-α'disubstitué ayant de 4 à 12 atomes de carbone, un radical cycloalkyle ayant de 3 à 12 atomes de carbone, un radical mono ou polycyclique ayant de 5 à 12 atomes de carbone lié au noyau phényle par un carbone tertiaire, R₅ et R₇ ne pouvant représenter simultanément OH ou alcoxy,
R₆ représente un atome d'hydrogène, OH, un radical alkyle inférieur, un radical alkoxy ayant de 1 à 6 atomes de carbone, un radical cycloalkyle ayant de 3 à 12 atomes de carbone, un radical monohydroxyalkyle, un radical polyhydroxyalkyle, un atome de fluor, un atome de chlore, un radical alkényle ayant de 2 à 6 atomes de carbone ou un radical alkényloxy ayant de 2 à 6 atomes de carbone, R₄, R₅, R₆ et R₇ ne pouvant représenter simultanément un atome d'hydrogène,
R₅ et R₆ ou R₆ et R₇ pris ensemble peuvent former avec le cycle aromatique adjacent un cycle à 5 ou 6 chainons éventuellement substitué par des groupes méthyle et/ou éventuellement interrompu par un atome d'oxygène ou de soufre,
X est un radical divalent qui peut être lu de gauche à droite ou inversement choisi dans le groupe constitué par :
(i) -C(R₁₃R₁₄)-C(R₁₆R₁₈)-W-
(ii) -C(R₁₄R₁₆)-W-C(R₁₈R₁₉)-
(iii) -C(R₁₃R₁₄)-C(R₁₅R₁₆)-C(R₁₈R₂₀)-
(iv) -CR₁₇=CR₂₁-C(R₁₃R₁₄)-
dans lesquels :
W représente un atome d'oxygène, le groupe -NR₁₂ ou le groupe S(O)n, n étant 0, 1 ou 2,
R₁₃, R₁₅ et R₂₀ représentent un atome d'hydrogène, le radical -OR₁₁, -OCOR₁₁, -NHCOR₁₁, un radical 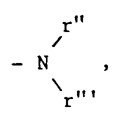 un radical aralkyle, un radical alkyle inférieur, un radical monohydroxyalkyle ou un radical polyhydroxyalkyle,
r'' et r''' identiques ou différents, représentant un atome d'hydrogène, un radical alkyle inférieur, un radical alkényle ayant de 2 à 6 atomes de carbone ou un radical alkynyle ayant de 2 à 6 atomes de carbone,
R₁₄, R₁₆, R₁₈ et R₁₉ représentent un atome d'hydrogène, un radical aralkyle, un radical alkyle inférieur, un radical monohydroxyalkyle ou polyhydroxyalkyle,
lorsque X représente (i), R₁₃ et R₁₄ peuvent former un groupe =N-OR₁₁ ou un groupe =N-OCOR₁₁,
lorsque X représente (iii) ou (iv), R₁₄, R₁₆ et R₁₈ peuvent également représenter le radical -OR₁₁ ou le radical -OCOR₁₁, ou encore R₁₃, R₁₄ ou R₁₅, R₁₆ pris ensemble peuvent former un groupe =NOR₁₁ ou un groupe =N-OCOR₁₁,
R₁₂ représentant un atome d'hydrogène, un radical alkyle inférieur, un radical aralkyle, un radical alkényle ayant de 2 à 6 atomes de carbone, un radical alkynyle ayant de 2 à 6 atomes de carbone ou un radical fluoroalkyle ayant de 1 à 6 atomes de carbone et de 3 à 7 atomes de fluor,
R₁₇ représentant un atome d'hydrogène, un groupe hydroxyle, un radical alkyle inférieur ou un radical alkoxy ayant de 1 à 6 atomes de carbone,
R₂₁ représentant un atome d'hydrogène ou un radical alkyle inférieur,
et les sels des composés de formule (I) lorsque R₁ représente une fonction acide carboxylique ou lorsque R₁₃, R₁₅ ou R₂₀ représente une fonction amine et les isomères optiques des composés de formule (I).

2. Composition cosmétique selon la revendication 1, caractérisée par le fait qu'elle contient le composé de formule (I) à une concentration comprise entre 0,001 et 3 % en poids.

3. Composition selon la revendication 1, caractérisée par le fait que le composé de formule (I) se présente sous forme de sels d'un métal alcalin ou alcalino-terreux ou encore de zinc ou d'une amine organique.

4. Composition selon la revendication 1, caractérisée par le fait que le composé de formule (I) se présente sous forme de sels d'un acide minéral ou organique choisi parmi l'acide chlorhydrique, sulfurique, acétique, citrique, fumarique, hémisuccinique, maléique et mandélique.

5. Composition selon l'une quelconque des revendications précédentes, caractérisée par le fait que le composé de formule (I) est pris dans le groupe constitué par :
Acide 2-hydroxy-4-[2-hydroxy-2-(5,6,7,8-tétrahydro-5,5,8,8-tétraméthyl-2-naphtyl)éthoxy]benzoïque ;
2-hydroxy-4-[2-hydroxy-2-(5,6,7,8-tétrahydro-5,5,8,8-tétraméthyl-2-naphtyl)éthoxy]benzoate de méthyle ;
Acide 2-hydroxy-4-[2-hydroxyimino-2-(5,6,7,8-tétrahydro-5,5,8,8-tétraméthyl-2-naphtyl)éthoxy]benzoïque ;
Acide 2-acétyloxy-4-[2-acétyloxy-2-(5,6,7,8-tétrahydro-5,5,8,8-tétraméthyl-2-naphtyl)éthoxy]benzoïque ;
Acide 2-hydroxy-4-[2-acétyloxy-2-(5,6,7,8-tétrahydro-5,5,8,8-tétraméthyl-2-naphtyl)éthoxy]benzoïque ;
Acide 2-acétyloxy-4-[2-hydroxy-2-(5,6,7,8-tétrahydro-5,5,8,8-tétraméthyl-2-napthyl)éthoxy]benzoïque ;
Acide-2-hydroxy-4-[2-(5,6,7,8-tétrahydro-5,5,8,8-tétraméthyl -2-naphtyl)éthoxy]benzoïque ;
2-hydroxy-4- [2-hydroxy-2-(5,6,7,8-tétrahydro-5,5,8,8-tétraméthyl-2-naphtyl)éthoxy]benzyl alcool ;
Acétate de 2-acétyloxy-4-[2-hydroxy-2-(5,6,7,8-tétrahydro-5,5,8,8-tétraméthyl-2-naphtyl)éthoxy]benzyl alcool ;
Piperidinylamide de l' acide 2-hydroxy-4-[2-hydroxy-2-(5,6,7,8-tétrahydro-5,5,8,8-tétraméthyl-2-naphtyl)éthoxy]benzoïque ;
Morpholinylamide de l'acide 2-hydroxy-4-[2-hydroxy-2-(5,6,7,8-tétrahydro-5,5,8,8-tétraméthyl-2-naphtyl)éthoxy]benzoïque.
2-hydroxy-4-[2-hydroxy-2-(5,6,7,8-tétrahydro-5,5,8,8-tétraméthyl-2-naphtyl)éthoxy]benzamide ;
N-éthyl amide de l'acide 2-hydroxy-4-[2-hydroxy-2-(5,6,7,8-tétrahydro-5,5,8,8-tétraméthyl-2-naphtyl)éthoxy]benzoïque ;
2-hydroxy-4-[2-hydroxy-2-(4,4-diméthylthiochroman-6-yl) éthoxy]benzoate de méthyle ;
Acide 2-hydroxy-4-[2-hydroxy-2-(4,4-diméthylthiochroman-6-yl) éthoxy]benzoïque ;
Acide 2-hydroxy-4-[3-hydroxy-3-(5,6,7,8-tétrahydro-5,5,8,8-tétraméthyl-2-naphtyl)propyl]benzoïque ;
Acide 2-hydroxy-4-[2-hydroxy-2-(3,5-di-tert-butyl-4-hydroxyphényl)éthoxy]benzoïque ;
2-hydroxy-4-[2-hydroxy-2-(5,6,7,8-tétrahydro-5,5,8,8-tétraméthyl-2-naphtyl)éthoxy]toluène ;
2,6-dihydroxy-4-[2-hydroxy-2-(5,6,7,8-tétrahydro-5,5,8,8-tétraméthyl-2-naphtyl)éthoxy]benzoate de méthyle
Acide 2-hydroxy-4-[2-hydroxy-2-(3-tert-butyl-4-méthoxyphényl) éthoxy]benzoïque ;
Acide 2-hydroxy-4-[2-hydroxy-2-(3-tert-butyl-4-hydroxyphényl) éthoxy]benzoïque ;
Isomère (-) de l'acide 2-hydroxy-4-[2-hydroxy-2-(5,6,7,8-tétrahydro-5,5,8,8-tétraméthyl-2-naphtyl)éthoxy]benzoïque ;
Isomère (+) de l'acide 2-hydroxy-4-[2-hydroxy-2-(5,6,7,8-tétrahydro-5,5,8-8-tétraméthyl-2-naphtyl)éthoxy]benzoïque ;
Acide 2-hydroxy-4-[2-hydroxy-2(3-méthoxy-5,6,7,8-tétrahydro-5,5,8,8-tétraméthyl-2-naphtyl)éthoxy]benzoïque ;
Acide 2-méthoxy-4-[2-hydroxy-2-(5,6,7,8-tétrahydro-5,5,8,8-tétraméthyl-2-naphtyl)éthoxy]benzoïque ;
Acide 2-hydroxy-4-[2-hydroxy-2-(3-hydroxy-5,6,7,8-tétrahydro-5,5,8,8-tétraméthyl-2-naphtyl)éthoxy]benzoïque ;
Acide 2-hydroxy-4-[2-amino-2-(5,6,7,8-tétrahydro-5,5,8,8-tétraméthyl-2-naphtyl)éthoxy]benzoïque ;
Acide 2-hydroxy-4-[2-hydroxy-2-(5,6,7,8-tétrahydro-5,5,8,8-tétraméthyl-2-naphtyl)propyloxy]benzoïque ;
Acide 2-hydroxy-4-[2-hydroxy-2-(5,6,7,8-tétrahydro-5,5,8,8-tétraméthyl-2-naphtyl)hexyloxy]benzoïque ;
Acide 2-hydroxy-4-[2-hydroxy-2-(5,6,7,8-tétrahydro-5,5,8,8-tétraméthyl-2-naphtyl)éthylamino]benzoïque ;
Acide 2-hydroxy-4-[[2-hydroxy-2-[3-(1-adamantyl)-4-méthoxyphényl] éthoxy]]benzoïque ;
Acide 2-hydroxy-4-[[2-[3-(1-adamantyl)-4-méthoxyphenyl] éthoxy]]benzoïque.

## Claims (Claims for the following Contracting State(s): AT, BE, CH, LI, DE, DK, FR, GB, GR, IT, NL, PT, SE)

1. Biaromatic compounds, characterized in that they correspond to the following general formula: 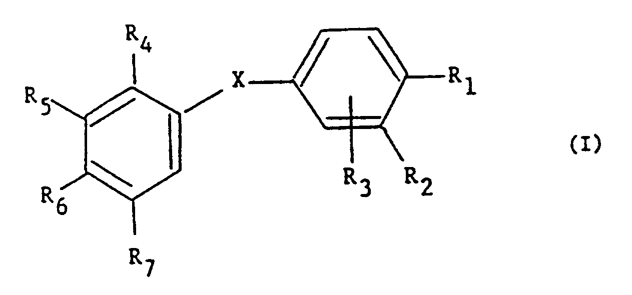 in which:
R₁ represents the -CH₃ radical, the -CH₂OH radical, the -COR₈ radical or the -CH₂OCOR₉ radical,
R₈ representing a hydrogen atom, OH, -OR₁₀, 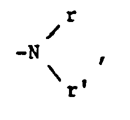 or a lower alkyl radical,
R₁₀ representing an alkyl radical having from 1 to 20 carbon atoms, an alkenyl radical having from 2 to 20 carbon atoms or an aryl or aralkyl radical,
r and r', which are identical or different, representing a hydrogen atom, a lower alkyl radical, an aryl radical, an aralkyl radical, an α-amino acid residue, a sugar residue or a heterocycle or r and r', taken together, form a heterocycle,
R₉ representing an alkyl radical having from 1 to 20 carbon atoms, an alkenyl radical having from 2 to 20 carbon atoms or a sugar residue,
R₂ and R₃ represent -OR₁₁ or -OCOR₁₁
R₁₁ representing a hydrogen atom, a lower alkyl radical, a fluoroalkyl radical having from 1 to 6 carbon atoms and from 3 to 7 fluorine atoms, an aryl radical or an aralkyl radical,
it additionally being possible for R₃ to represent a hydrogen atom,
R₄ represents a hydrogen atom, OH, a lower alkyl radical, an alkoxy radical having from 1 to 6 carbon atoms, a fluorine or chlorine atom or the -CF₃ group,
R₅ and R₇ represent a hydrogen atom, OH, an alkoxy radical having from 1 to 6 carbon atoms, an α-substituted alkyl radical having from 3 to 12 carbon atoms or an α,α'-disubstituted alkyl radical having from 4 to 12 carbon atoms, a cycloalkyl radical having from 3 to 12 carbon atoms or a mono- or polycyclic radical having from 5 to 12 carbon atoms bonded to the phenyl ring via a tertiary carbon, it not being possible for R₅ and R₇ simultaneously to represent OH or alkoxy,
R₆ represents a hydrogen atom, OH, a lower alkyl radical, an alkoxy radical having from 1 to 6 carbon atoms, a cycloalkyl radical having from 3 to 12 carbon atoms, a monohydroxyalkyl radical, a polyhydroxyalkyl radical, a fluorine atom, a chlorine atom, an alkenyl radical having from 2 to 6 carbon atoms or an alkenyloxy radical having from 2 to 6 carbon atoms, it not being possible for R₄, R₅, R₆ and R₇ simultaneously to represent a hydrogen atom,
R₅ and R₆ or R₆ and R₇, taken together, can form, with the adjacent aromatic ring, a 5- or 6-membered ring optionally substituted by methyl groups and/or optionally interrupted by an oxygen or sulphur atom,
X is a divalent radical, which can be read from left to right or vice versa, chosen from the group consisting of:
(i) -C(R₁₃R₁₄)-C(R₁₆R₁₈)-W-
(ii) -C(R₁₄R₁₆)-W-C(R₁₈R₁₉)-
(iii) -C(R₁₃R₁₄)-C(R₁₅R₁₆)-C(R₁₈R₂₀)-
(iv) -CR₁₇=CR₂₁-C(R₁₃R₁₄)-
in which:
W represents an oxygen atom, the -NR₁₂ group or the S(O)ₙ group, n being 0, 1 or 2,
R₁₃, R₁₅ and R₂₀ represent a hydrogen atom, the -OR₁₁, -OCOR₁₁ or -NHCOR₁₁ radical, a 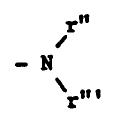 radical, an aralkyl radical, a lower alkyl radical, a monohydroxyalkyl radical or a polyhydroxyalkyl radical,
r'' and r''', which are identical or different, representing a hydrogen atom, a lower alkyl radical, an alkenyl radical having from 2 to 6 carbon atoms or an alkynyl radical having from 2 to 6 carbon atoms,
R₁₄, R₁₆, R₁₈ and R₁₉ represent a hydrogen atom, an aralkyl radical, a lower alkyl radical or a monohydroxyalkyl or polyhydroxyalkyl radical,
when X represents (i), R₁₃ and R₁₄ can form an =N-OR₁₁ group or an =N-OCOR₁₁ group,
when X represents (iii) or (iv), R₁₄, R₁₆ and R₁₈ can also represent the -OR₁₁ radical or the -OCOR₁₁ radical, or alternatively R₁₃ and R₁₄ or R₁₅ and R₁₆, taken together, can form an =NOR₁₁ group or an =N-OCOR₁₁ group,
R₁₂ representing a hydrogen atom, a lower alkyl radical, an aralkyl radical, an alkenyl radical having from 2 to 6 carbon atoms, an alkynyl radical having from 2 to 6 carbon atoms or a fluoroalkyl radical having from 1 to 6 carbon atoms and from 3 to 7 fluorine atoms,
R₁₇ representing a hydrogen atom, a hydroxyl group, a lower alkyl radical or an alkoxy radical having from 1 to 6 carbon atoms,
R₂₁ representing a hydrogen atom or a lower alkyl radical,
and the salts of the compounds of formula (I) when R₁ represents a carboxylic acid functional group or when R₁₃, R₁₅ or R₂₀ represents an amine functional group and the optical isomers of the compounds of formula (I).

2. Compounds according to Claim 1, characterized in that they are provided in the form of salts of an alkali metal or alkaline-earth metal or alternatively of zinc or of an organic amine.

3. Compounds according to Claim 1, characterized in that they are provided in the form of salts of an inorganic or organic acid chosen from hydrochloric, sulphuric, acetic, citric, fumaric, hemisuccinic, maleic and mandelic acid.

4. Compounds according to Claim 1, characterized in that the lower alkyl radical having from 1 to 6 carbon atoms is taken from the group consisting of the methyl, ethyl, isopropyl, butyl and tert-butyl radicals.

5. Compounds according to Claim 1, characterized in that the alkoxy radical having from 1 to 6 carbon atoms is taken from the group consisting of a methoxy, ethoxy, isopropoxy and butoxy radical.

6. Compounds according to Claim 1, characterized in that the α,α'-disubstituted alkyl radical is taken from the group consisting of: a tert-butyl, 1,1-dimethylpropyl, 1-methyl-1-ethylpropyl, 1-methyl-1-ethylhexyl and 1,1-dimethyldecyl radical.

7. Compounds according to Claim 1, characterized in that the mono- or polycyclic cycloalkyl radical having from 5 to 12 carbon atoms in which the bonding carbon is trisubstituted is the 1-methylcyclohexyl or 1-adamantyl radical.

8. Compounds according to Claim 1, characterized in that the monohydroxyalkyl radical is the 2-hydroxyethyl, 2-hydroxypropyl or 3-hydroxypropyl radical.

9. Compounds according to Claim 1, characterized in that the polyhydroxyalkyl radical contains from 2 to 6 carbon atoms and from 2 to 5 hydroxyl groups and is taken from the group consisting of the 2,3-dihydroxypropyl, 2,3,4-trihydroxybutyl and 2,3,4,5-tetrahydroxypentyl radical and the pentaerythritol residue.

10. Compounds according to Claim 1, characterized in that the aryl radical is a phenyl radical optionally substituted by at least one halogen atom, one hydroxyl or one nitro functional group.

11. Compounds according to Claim 1, characterized in that the aralkyl radical is the benzyl or phenethyl radical optionally substituted by at least one halogen atom, one hydroxyl or one nitro functional group.

12. Compounds according to Claim 1, characterized in that the alkynyl radical having from 2 to 6 carbon atoms is the propargyl radical.

13. Compounds according to Claim 1, characterized in that the alkenyl radical having from 2 to 6 carbon atoms is taken from the group consisting of the vinyl, allyl and 2-butenyl radical.

14. Compounds according to Claim 1, characterized in that the lower fluoroalkyl radical is a radical having from 1 to 6 carbon atoms and from 3 to 7 fluorine atoms.

15. Compounds according to Claim 1, characterized in that the heterocycle is taken from the group consisting of a piperidino, morpholino, pyrrolidino or piperazino radical, optionally substituted in the 4-position by a mono- or polyhydroxyalkyl or C₁-C₆ alkyl radical.

16. Compounds according to any one of the preceding claims, characterized in that they are taken from the group consisting of:
2-Hydroxy-4-[2-hydroxy-2-(5,6,7,8-tetrahydro-5,5,8,8-tetramethyl-2-naphthyl)ethoxy]benzoic acid;
Methyl 2-hydroxy-4-[2-hydroxy-2-(5,6,7,8-tetrahydro-5,5,8,8-tetramethyl-2-naphthyl)ethoxy]benzoate;
2-Hydroxy-4-[2-hydroxyimino-2-(5,6,7,8-tetrahydro-5,5,8,8-tetramethyl-2-naphthyl)ethoxy]benzoic acid;
2-Acetyloxy-4-[2-acetyloxy-2-(5,6,7,8-tetrahydro-5,5,8,8-tetramethyl-2-naphthyl)ethoxy]benzoic acid;
2-Hydroxy-4-[2-acetyloxy-2-(5,6,7,8-tetrahydro-5,5,8,8-tetramethyl-2-naphthyl)ethoxy]benzoic acid;
2-Acetyloxy-4-[2-hydroxy-2-(5,6,7,8-tetrahydro-5,5,8,8-tetramethyl-2-naphthyl)ethoxy]benzoic acid;
2-Hydroxy-4-[2-(5,6,7,8-tetrahydro-5,5,8,8-tetramethyl-2-naphthyl)ethoxy]benzoic acid;
2-Hydroxy-4-[2-hydroxy-2-(5,6,7,8-tetrahydro-5,5,8,8-tetramethyl-2-naphthyl)ethoxy]benzyl alcohol;
2-Acetyloxy-4-[2-hydroxy-2-(5,6,7,8-tetrahydro-5,5,8,8-tetramethyl-2-naphthyl)ethoxy]benzyl alcohol acetate;
Piperidinylamide of 2-hydroxy-4-[2-hydroxy-2-(5,6,7,8-tetrahydro-5,5,8,8-tetramethyl-2-naphthyl)-ethoxy]benzoic acid;
Morpholinylamide of 2-hydroxy-4-[2-hydroxy-2-(5,6,7,8-tetrahydro-5,5,8,8-tetramethyl-2-naphthyl)-ethoxy]benzoic acid;
2-Hydroxy-4-[2-hydroxy-2-(5,6,7,8-tetrahydro-5,5,8,8-tetramethyl-2-naphthyl)ethoxy]benzamide;
N-Ethylamide of 2-hydroxy-4-[2-hydroxy-2-(5,6,7,8-tetrahydro-5,5,8,8-tetramethyl-2-naphthyl)-ethoxy]benzoic acid;
Methyl 2-hydroxy-4-[2-hydroxy-2-(4,4-dimethyl-thiochroman-6-yl)ethoxy]benzoate;
2-Hydroxy-4-[2-hydroxy-2-(4,4-dimethylthio-chroman-6-yl)ethoxy]benzoic acid;
2-Hydroxy-4-[3-hydroxy-3-(5,6,7,8-tetrahydro-5,5,8,8-tetramethyl-2-naphthyl)propyl]benzoic acid;
2-Hydroxy-4-[2-hydroxy-2-(3,5-di-tert-butyl-4-hydroxyphenyl)ethoxy]benzoic acid;
2-Hydroxy-4-[2-hydroxy-2-(5,6,7,8-tetrahydro-5,5,8,8-tetramethyl-2-naphthyl)ethoxy]toluene;
Methyl 2,6-dihydroxy-4-[2-hydroxy-2-(5,6,7,8-tetrahydro-5,5,8,8-tetramethyl-2-naphthyl)ethoxy]-benzoate;
2-Hydroxy-4-[2-hydroxy-2-(3-tert-butyl-4-methoxyphenyl)ethoxy]benzoic acid;
2-Hydroxy-4-[2-hydroxy-2-(3-tert-butyl-4-hydroxyphenyl)ethoxy]benzoic acid;
(-)Isomer of 2-hydroxy-4-[2-hydroxy-2-(5,6,7,8-tetrahydro-5,5,8,8-tetramethyl-2-naphthyl)ethoxy]benzoic acid;
(+)Isomer of 2-hydroxy-4-[2-hydroxy-2-(5,6,7,8-tetrahydro-5,5,8,8-tetramethyl-2-naphthyl)ethoxy]benzoic acid;
2-Hydroxy-4-[2-hydroxy-2-(3-methoxy-5,6,7,8-tetrahydro-5,5,8,8-tetramethyl-2-naphthyl)ethoxy]benzoic acid;
2-Methoxy-4-[2-hydroxy-2-(5,6,7,8-tetrahydro-5,5,8,8-tetramethyl-2-naphthyl)ethoxy]benzoic acid;
2-Hydroxy-4-[2-hydroxy-2-(3-hydroxy-5,6,7,8-tetrahydro-5,5,8,8-tetramethyl-2-naphthyl)ethoxy]benzoic acid;
2-Hydroxy-4-[2-amino-2-(5,6,7,8-tetrahydro-5,5,8,8-tetramethyl-2-naphthyl)ethoxy]benzoic acid;
2-Hydroxy-4-[2-hydroxy-2-(5,6,7,8-tetrahydro-5,5,8,8-tetramethyl-2-naphthyl)propyloxy]benzoic acid;
2-Hydroxy-4-[2-hydroxy-2-(5,6,7,8-tetrahydro-5,5,8,8-tetramethyl-2-naphthyl)hexyloxy]benzoic acid;
2-Hydroxy-4-[2-hydroxy-2-(5,6,7,8-tetrahydro-5,5,8,8-tetramethyl-2-naphthyl)ethylamino]benzoic acid;
2-Hydroxy-4-[2-hydroxy-2-[3-(1-adamantyl)-4-methoxyphenyl]ethoxy]benzoic acid;
2-Hydroxy-4-[2-[3-(1-adamantyl)-4-methoxyphenyl]-ethoxy]benzoic acid.

17. Pharmaceutical composition, characterized in that it contains, in an appropriate vehicle for enteral, parenteral, topical or ocular administration, at least one compound of formula (I) according to any one of Claims 1 to 16.

18. Composition according to Claim 17, characterized in that it contains from 0.001 to approximately 5 % by weight of a compound of formula (I).

19. Use of a compound according to any one of Claims 1 to 16 for the preparation of a pharmaceutical composition intended for the treatment of dermatological, rheumatic, respiratory and ophthalmological conditions.

20. Cosmetic composition for body and hair hygiene, characterized in that it contains, in an appropriate cosmetic vehicle, at least one compound of formula (I) according to any one of Claims 1 to 16.

21. Cosmetic composition according to Claim 20, characterized in that it contains the compound of formula (I) at a concentration of between 0.001 and 3 % by weight.

## Claims (Claims for the following Contracting State(s): ES)

1. Cosmetic composition for body and hair hygiene, characterized in that it contains, in an appropriate cosmetic vehicle, at least one compound of formula (I): 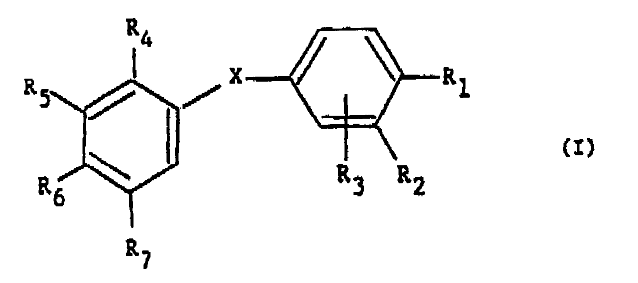 in which:
R₁ represents the -CH₃ radical, the -CH₂OH radical, the -COR₈ radical or the -CH₂OCOR₉ radical,
R₈ representing a hydrogen atom, OH, -OR₁₀, 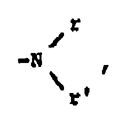 or a lower alkyl radical,
R₁₀ representing an alkyl radical having from 1 to 20 carbon atoms, on alkenyl radical having from 2 to 20 carbon atoms or an aryl or aralkyl radical,
r and r', which are identical or different, representing a hydrogen atom, a lower alkyl radical, an aryl radical, an aralkyl radical, an α-amino acid residue, a sugar residue or a heterocycle or r and r', taken together, form a heterocycle,
R₉ representing an alkyl radical having from 1 to 20 carbon atoms, an alkenyl radical having from 2 to 20 carbon atoms or a sugar residue,
R₂ and R₃ represent -OR₁₁ or -OCOR₁₁
R₁₁ representing a hydrogen atom, a lower alkyl radical, a fluoroalkyl radical having from 1 to 6 carbon atoms and from 3 to 7 fluorine atoms, an aryl radical or an aralkyl radical,
it additionally being possible for R₃ to represent a hydrogen atom,
R₄ represents a hydrogen atom, OH, a lower alkyl radical, an alkoxy, radical having from 1 to 6 carbon atoms, a fluorine or chlorine atom or the -CF₃ group,
R₅ and R₇ represent a hydrogen atom, OH, an alkoxy radical having from 1 to 6 carbon atoms, an α-substituted alkyl radical having from 3 to 12 carbon atoms or an α,α'-disubstituted alkyl radical having from 4 to 12 carbon atoms, a cycloalkyl radical having from 3 to 12 carbon atoms or a mono- or polycyclic radical having from 5 to 12 carbon atoms bonded to the phenyl ring via a tertiary carbon, it not being possible for R₅ and R₇ simultaneously to represent OH or alkoxy,
R₆ represents a hydrogen atom, OH, a lower alkyl radical, an alkoxy radical having from 1 to 6 carbon atoms, a cycloalkyl radical having from 3 to 12 carbon atoms, a monohydxoxyalkyl radical, a polyhydroxyalkyl radical, a fluorine atom, a chlorine atom, an alkenyl radical having from 2 to 6 carbon atoms or an alkenyloxy radical having from 2 to 6 carbon atoms, it not being possible for R₄, R₅, R₆ and R₇ simultaneously to represent a hydrogen atom,
R₅ and R₆ or R₆ and R₇, taken together, can form, with the adjacent aromatic ring, a 5- or 6-membered ring optionally substituted by methyl groups and/or optionally interrupted by an oxygen or sulphur atom,
X is a divalent radical, which can be read from left to right or vice versa, chosen from the group consisting of:
(i) -C(R₁₃R₁₄)-C(R₁₆R₁₈)-W-
(ii) -C(R₁₄R₁₆)-W-C(R₁₈R₁₉)-
(iii) -C(R₁₃R₁₄)-C(R₁₅R₁₆)-C(R₁₈R₂₀)-
(iv) -CR₁₇=CR₂₁-C(R₁₃R₁₄)-
in which:
W represent: an oxygen atom, the -NR₁₂ group or the S(O)ₙ group, n being 0, 1 or 2,
R₁₃, R₁₅ and R₂₀ represent a hydrogen atom, the -OR₁₁, -OCOR₁₁ or -NHCOR₁₁ radical, a 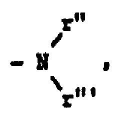 radical, an aralkyl radical, a lower alkyl radical, a monohydroxyalkyl radical or a polyhydroxyalkyl radical,
r'' and r''', which are identical or different, representing a hydrogen atom, a lower alkyl radical, an alkenyl radical having from 2 to 6 carbon atoms or an alkynyl radical having from 2 to 6 carbon atoms,
R₁₄, R₁₆, R₁₈, and R₁₉ represent a hydrogen atom, an aralkyl radical, a lower alkyl radical or a monohydroxyalkyl or polyhydroxyalkyl radical,
when X represent: (i), R₁₃ and R₁₄ can form an =N-OR₁₁ group or an =N-OCOR₁₁ group,
when X represents (iii) or (iv), R₁₄, R₁₆ and R₁₈ can also represent the -OR₁₁ radical or the -OCOR₁₁ radical, or alternatively R₁₃ and R₁₄ or R₁₅ and R₁₆, taken together, can form in =NOR₁₁ group or an =N-OCOR₁₁ group,
R₁₂ representing a hydrogen atom, a lower alkyl radical, an aralkyl radical, an alkenyl radical having from 2 to 6 carbon atoms, an alkynyl radical having from 2 to 6 carbon atoms or a fluoroalkyl radical having from 1 to 6 carbon atoms and from 3 to 7 fluorine atoms,
R₁₇ representing a hydrogen atom, a hydroxyl group, a lower alkyl radical or an alkoxy radical having from 1 to 6 carbon atoms,
R₂₁ representing a hydrogen atom or a lower alkyl radical,
and the salts of the compounds of formula (I) when R₁ represents a carboxylic acid functional group or when R₁₃, R₁₅ or R₂₀ represents an amine functional group and the optical isomers of the compounds of formula (I).

2. Cosmetic composition according to Claim 1, characterized in that it contains the compound of formula (I) at a concentration of between 0.001 and 3 % by weight.

3. Composition according to Claim 1, characterized in that the compound of formula (I) is provided in the form of salts of an alkali metal or alkaline-earth metal or alternatively of zinc or of an organic amine.

4. Composition according to Claim 1, characterized in that the compound of formula (I) is provided in the form of salts of an inorganic or organic acid chosen from hydrochloric, sulphuric, acetic, citric, fumaric, hemisuccinic, maleic and mandelic acid.

5. Composition according to any one of the preceding claims, characterized in that the compound of formula (I) is taken from the group consisting of:
2-Hydroxy-4-[2-hydroxy-2-(5,6,7,8-tetrahydro-5,5,8,8-tetramethyl-2-naphthyl)ethoxy]benzoic acid;
Methyl 2-hydroxy-4-[2-hydroxy-2-(5,6,7,8-tetrahydro-5,5,8,8-tetramethyl-2-naphthyl)ethoxy]benzoate;
2-Hydroxy-4-[2-hydroxyimino-2-(5,6,7,8-tetrahydro-5,5,8,8-tetramethyl-2-naphthyl)ethoxy]benzoic acid;
2-Acetyloxy-4-[2-acetyloxy-2-(5,6,7,8-tetrahydro-5,5,8,8-tetramethyl-2-naphthyl)ethoxy]benzoic acid;
2-Hydroxy-4-[2-acetyloxy-2-(5,6,7,8-tetrahydro-5,5,8,8-tetramathyl-2-naphthyl)ethoxy]benzoic acid;
2-Acetyloxy-4-[2-hydroxy-2-(5,6,7,8-tetrehydro-5,5,8,8-tetramethyl-2-naphthyl)ethoxy]benzoic acid;
2-Hydroxy-4-[2-(5,6,7,8-tetrahydro-5,5,8,8-tetramethyl-2-naphthyl)ethoxy]benzoic acid;
2-Hydroxy-4-[2-hydroxy-2-(5,6,7,8-tetrahydro-5,5,8,8-tetramethyl-2-naphthyl)ethoxy]benzyl alcohol;
2-Acetyloxy-4-[2-hydroxy-2-(5,6,7,8-tetrahydro-5,5,8,8-tetramethyl-2-naphthyl)ethoxy]benzyl alcohol acetate;
Piperidinylamide of 2-hydroxy-4-[2-hydroxy-2-(5,6,7,8-tetrahydro-5,5,8,8-tetramethyl-2-naphthyl)-ethoxy]benzoic acid;
Morpholinylamide of 2-hydroxy-4-[2-hydroxy-2-(5,6,7,8-tetrahydro-5,5,8,8-tetramethyl-2-naphthyl)- ethoxy]benzoic acid;
2-Hydroxy-4-[2-hydroxy-2-(5,6,7,8-tetrahydro-5,5,8,8-tetramethyl-2-naphthyl)ethoxy]benzamide;
N-Ethylamide of 2-hydroxy-4-[2-hydroxy-2-(5,6,7,8-tetrahydro-5,5,8,8-tetramethyl-2-naphthyl)-ethoxy]benzoic acid;
Methyl 2-hydroxy-4-[2-hydroxy-2-(4,4-dimethyl-thiochroman-6-yl)ethoxy]benzoate;
2-Hydroxy-4-[2-hydroxy-2-(4,4-dimethylthiochroman-6-yl)ethoxy]benzoic acid;
2-Hydroxy-4-[3-hydroxy-3-(5,6,7,8-tetrahydro-5,5,8,8-tetrmethyl-2-naphthyl)propyl]benzoic acid;
2-Hydroxy-4-[2-hydroxy-2-(3,5-di-tert-butyl-4-hydroxyphenyl)ethoxy]benzoic acid;
2-Hydroxy-4-[2-hydroxy-2-(5,6,7,8-tetrahydro-5,5,8,8-tetramethyl-2-naphthyl)ethoxy]toluene;
Methyl 2,6-dihydroxy-4-[2-hydroxy-2-(5,6,7,8-tetrahydro-5,5,8,8-tetramethyl-2-naphthyl)ethoxy]-benzoate;
2-Hydroxy-4-[2-hydroxy-2-(3-tert-butyl-4-methoxyphenyl)ethoxy]benzoic acid;
2-Hydroxy-4-[2-hydroxy-2-(3-tert-butyl-4-hydroxy-phenyl)ethoxy]benzoic acid;
(-)Isomer of 2-hydroxy-4-[2-hydroxy-2-(5,6,7,8-tetrahydro-5,5,8,8-tetramethyl-2-naphthyl)ethoxy]benzoic acid;
(+)Isomer of 2-hydroxy-4-[2-hydroxy-2-(5,6,7,8-tetrahydro-5,5,8,8-tetramethyl-2-naphthyl)ethoxy]benzoic acid;
2-Hydroxy-4-[2-hydroxy-2-(3-methoxy-5,6,7,8-tetrahydro-5,5,8,8-tetramethyl-2-naphthyl)ethoxy]benzoic acid;
2-Methoxy-4-[2-hydroxy-2-(5,6,7,8-tetrahydro-5,5,8,8-tetramethyl-2-naphthyl)ethoxy]benzoic acid;
2-Hydroxy-4-[2-hydroxy-2-(3-hydroxy-5,6,7,8-tetrahydro-5,5,8,8-tetramethyl-2-naphthyl)ethoxy]benzoic acid;
2-Hydroxy-4-[2-amino-2-(5,6,7,8-tetrahydro-5,5,8,8-tetramethyl-2-naphthyl)ethoxy]benzoic acid;
2-Hydroxy-4-[2-hydroxy-2-(5,6,7,8-tetrahydro-5,5,8,8-tetramethyl-2-naphthyl)propyloxy]benzoic acid;
2-Hydroxy-4-[2-hydroxy-2-(5,6,7,8-tetrahydro-5,5,8,8-tetramethyl-2-naphthyl)hexyloxy]benzoic acid;
2-Hydroxy-4-[2-hydroxy-2-(5,6,7,8-tetrahydro-5,5,8,8-tetramethyl-2-naphthyl)ethylamino]benzoic acid;
2-Hydroxy-4-[2-hydroxy-2-[3-(1-adamantyl)-4-methoxyphenyl]ethoxy]benzoic acid;
2-Hydroxy-4-[2-[3-(1-adamantyl)-4-methoxyphenyl]-ethoxy]benzoic acid.

## Patentansprüche (Patentansprüche für folgende(n) Vertragsstaat(en): AT, BE, CH, LI, DE, DK, FR, GB, GR, IT, NL, PT, SE)

1. Diaromatische Verbindungen, dadurch gekennzeichnet, daß sie der folgenden allgemeinen Formel entsprechen: 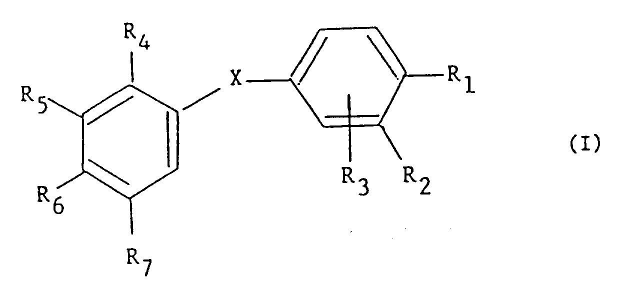 worin
R₁ die -CH₃-Gruppe, die -CH₂OH-Gruppe, den Rest -COR₈ oder den Rest -CH₂OCOR₉ bedeutet,
wobei R₈ ein Wasserstoffatom, OH, -OR₁₀, 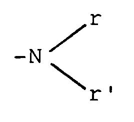 oder einen niedrigen Alkylrest bedeutet,
R₁₀ einen Alkylrest mit 1 bis 20 Kohlenstoffatomen, einen Alkenylrest mit 2 bis 20 Kohlenstoffatomen, einen Aryl- oder Aralkylrest darstellt,
r und r', die gleich oder verschieden sein können, ein Wasserstoffatom, einen niedrigen Alkylrest, einen Arylrest, einen d'α-Aminosäurerest, einen Zuckerrest oder einen Heterocyclus bedeuten, oder r und r' zusammengenommen einen Heterocyclus bilden,
R₉ einen Alkylrest mit 1 bis 20 Kohlenstoffatomen, einen Alkenylrest mit 2 bis 20 Kohlenstoffatomen oder einen Zuckerrest darstellt,
R₂ und R₃ -OR₁₁ oder -OCOR₁₁ bedeuten,
wobei R₁₁ ein Wasserstoffatom, einen niedrigen Alkylrest, einen Fluoralkylrest mit 1 bis 6 Kohlenstoffatomen und 3 bis 7 Fluoratomen, einen Aryl- oder Aralkylrest bedeutet,
R₃ weiterhin ein Wasserstoffatom bedeutet,
R₄ ein Wasserstoffatom, OH, einen niedrigen Alkylrest, einen Alkoxyrest mit 1 bis 6 Kohlenstoffatomen, ein Fluoratom, ein Chloratom oder die CF₃-Gruppe darstellt,
R₅ und R₇ ein Wasserstoffatom, OH, einen Alkoxyrest mit 1 bis 6 Kohlenstoffatomen, einen α-substituierten Alkylrest mit 3 bis 12 Kohlenstoffatomen oder einen α,α'-disubstituierten Alkylrest mit 4 bis 12 Kohlenstoffatomen, einen Cycloalkylrest mit 3 bis 12 Kohlenstoffatomen, einen mono- oder polycyclischen Rest mit 5 bis 12 Kohlenstoffatomen darstellen, der mit dem Phenylkern über ein tertiäres Kohlenstoffatom verknüpft ist, wobei R₅ und R₇ nicht gleichzeitig OH oder einen Alkoxyrest bedeuten können,
R₆ ein Wasserstoffatom, OH, einen niedrigen Alkylrest, einen Alkoxyrest mit 1 bis 6 Kohlenstoffatomen, einen Cycloalkylrest mit 3 bis 12 Kohlenstoffatomen, einen Monohydroxyalkylrest, einen Polyhydroxyalkylrest, ein Fluoratom, ein Chloratom, einen Alkenylrest mit 2 bis 6 Kohlenstoffatomen oder einen Alkenyloxyrest mit 2 bis 6 Kohlenstoffatomen darstellt, wobei R₄, R₅, R₆ und R₇ nicht gleichzeitig ein Wasserstoffatom bedeuten können, und
R₅ und R₆ oder R₆ und R₇ zusammengenommen mit dem benachbarten aromatischen Ring einen 5- oder 6-gliedrigen Ring bilden können, der gegebenenfalls durch Methylgruppen substituiert und/oder gegebenenfalls durch ein Sauerstoff- oder Schwefelatom unterbrochen sein kann,
X ein zweiwertiges Radial darstellt, welches von links nach rechts gelesen oder umgekehrt aus der Gruppe ausgewählt ist, die aus den folgenden Resten besteht:
(i) -C(R₁₃R₁₄)-C(R₁₆R₁₈)-W-
(ii) -C(R₁₄R₁₆)-W-C(R₁₈R₁₉)-
(iii) -C(R₁₃R₁₄)-C(R₁₅R₁₆)-C(R₁₈R₂₀)-
(iv) -CR₁₇=CR₂₁-C(R₁₃R₁₄)-
worin
W ein Sauerstoffatom, die -NR₁₂-Gruppe oder die S(O)n-Gruppe bedeutet, wobei n entweder 0, 1 oder 2 bedeutet,
R₁₃, R₁₅ und R₂₀ ein Wasserstoffatom, den Rest -OR₁₁, -OCOR₁₁, -NHCOR₁₁, einen Rest 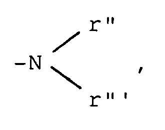 einen Aralkylrest, einen niedrigen Alkylrest, einen Monohydroxyalkylrest oder einen Polyhydroxyalkylrest bedeuten,
wobei r'' und r''', welche gleich oder verschieden sind, ein Wasserstoffatom, einen niedrigen Alkylrest, einen Alkenylrest mit 2 bis 6 Kohlenstoffatomen oder einen Alkinylrest mit 2 bis 6 Kohlenstoffatomen darstellen,
R₁₄, R₁₆, R₁₈ und R₁₉ ein Wasserstoffatom, einen Aralkylrest, einen niedrigen Alkylrest, einen Monohydroxyalkyl- oder Polyhydroxyalkylrest darstellen, wobei
unter der Maßgabe, daß X (i) bedeutet, R₁₃ und R₁₄ einen Rest =N-OR₁₁ oder einen Rest =N-OCOR₁₁ bilden können, und
unter der Maßgabe, daß X (iii) oder (iv) bedeutet, R₁₄, R₁₆ und R₁₈ jeweils den Rest -OR₁₁ oder den Rest OCOR₁₁ bedeuten können, oder R₁₃, R₁₄ oder R₁₅, R₁₆ zusammengenommen einen Rest =NOR₁₁ oder einen Rest =N-OCOR₁₁ bilden können,
wobei R₁₂ ein Wasserstoffatom, einen niedrigen Alkylrest, einen Aralkylrest, einen Alkenylrest mit 2 bis 6 Kohlenstoffatomen, einen Alkinylrest mit 2 bis 6 Kohlenstoffatomen oder einen Fluoralkylrest mit 1 bis 6 Kohlenstoffatomen und 3 bis 7 Fluoratomen darstellt,
R₁₇ ein Wasserstoffatom, eine Hydroxylgruppe, einen niedrigen Alkylrest oder einen Alkoxyrest mit 1 bis 6 Kohlenstoffatomen bedeutet,
R₂₁ ein Wasserstoffatom oder einen niedrigen Alkylrest bedeutet,
sowie die Salze der Verbindungen gemäß Formel (I) mit der Maßgabe, daß R₁ eine Carbonsäurefunktion bedeutet sowie mit der Maßgabe, daß R₁₃, R₁₅ oder R₂₀ eine Aminfunktion bedeutet sowie die optischen Isomeren von Verbindungen gemäß Formel (I).

2. Verbindungen gemäß Anspruch 1, dadurch gekennzeichnet, daß sie in Form der Alkalimetall- oder Erdalkalimetallsalze oder auch in Form der Zinksalze oder in Form eines organischen Amins vorliegen.

3. Verbindungen gemäß Anspruch 1, dadurch gekennzeichnet, daß sie in Form der Mineralsäuresalze oder in Form von Salzen organischer Säuren vorliegen, welche aus Salzsäure, Schwefelsäure, Essigsäure, Zitronensäure, Fumarsäure, Hemibernsteinsäure, Maleinsäure oder Mandelsäure ausgewählt sind.

4. Verbindungen gemäß Anspruch 1, dadurch gekennzeichnet, daß der niedrige Alkylrest 1 bis 6 Kohlenstoffatome aufweist und aus der Methyl-, Ethyl-, Isopropyl-, Butyl- und tert-Butylgruppe ausgewählt ist.

5. Verbindungen gemäß Anspruch 1, dadurch gekennzeichnet, daß der Alkoxyrest mit 1 bis 6 Kohlenstoffatomen aus der Methoxy-, Ethoxy-, Isopropoxy- und Butoxygruppe ausgewählt ist.

6. Verbindungen gemäß Anspruch 1, dadurch gekennzeichnet daß der α,α'-disubstituierte Alkylrest aus der Gruppe ausgewählt ist, die aus der aus der tert-Butyl-, 1,1-Dimethylpropyl-, 1-Methyl-1-ethylpropyl-, 1-Methyl-1-ethylhexyl- und 1,1-Dimethyldecylgruppe besteht.

7. Verbindungen gemäß Anspruch 1, dadurch gekennzeichnet, daß der mono- oder polycyclische Cycloalkylrest mit 5 bis 12 Kohlenstoffatomen, bei dem das Bindungskohlenstoffatom dreifach substituiert ist, die 1-Methylcyclohexyl- oder 1-Adamantylgruppe ist.

8. Verbindungen gemäß Anspruch 1, dadurch gekennzeichnet, daß der Monohydroxyalkylrest in Form der 2-Hydroxyethyl-, 2-Hydroxypropyl- oder 3-Hydroxypropylgruppe vorliegt.

9. Verbindungen gemäß Anspruch 1, dadurch gekennzeichnet, daß der Polyhydroxyalkylrest 2 bis 6 Kohlenstoffatome sowie 2 bis 5 Hydroxylgruppen aufweist und aus der Gruppe ausgewählt ist, die aus der 2,3-Dihydroxypropyl-, 2,3,4-Trihydroxybutyl-, 2,3,4,5-Tetrahydroxypentyl- und Pentaerythritgruppe besteht.

10. Verbindungen gemäß Anspruch 1, dadurch gekennzeichnet, daß der Arylrest eine gegebenenfalls durch mindestens ein Halogenatom, eine Hydroxylgruppe oder eine Nitrofunktion substituierte Phenylgruppe ist.

11. Verbindungen gemäß Anspruch 1, dadurch gekennzeichnet, daß der Aralkylrest eine gegebenenfalls durch mindestens ein Halogenatom, eine Hydroxylgruppe oder eine Nitrofunktion substituierte Benzyl- oder Phenethylgruppe darstellt.

12. Verbindungen gemäß Anspruch 1, dadurch gekennzeichnet, daß der Alkinylrest mit 2 bis 6 Kohlenstoffatomen die Propargylgruppe bedeutet.

13. Verbindungen gemäß Anspruch 1, dadurch gekennzeichnet, daß der Alkenylrest mit 2 bis 6 Kohlenstoffatomen aus der Gruppe ausgewählt ist, die aus der Vinyl-, Allyl- und 2-Butenylgruppe besteht.

14. Verbindungen gemäß Anspruch 1, dadurch gekennzeichnet, daß der niedrige Fluoralkylrest einen Rest mit 1 bis 6 Kohlenstoffatomen und 3 bis 7 Fluoratomen darstellt.

15. Verbindungen gemäß Anspruch 1, dadurch gekennzeichnet, daß der Heterocyclus aus der Gruppe ausgewählt ist, die aus Piperidin-, Morpholin-, Pyrrolidin- oder Piperazingruppe besteht, welche gegebenenfalls jeweils in der 4-Stellung durch einen C₁-C₆-Mono- oder -Polyhydroxyalkylrest substituiert ist.

16. Verbindungen gemäß einem der vorstehenden Ansprüche, dadurch gekennzeichnet, daß sie aus der Gruppe ausgewählt sind, die aus den folgenden Verbindungen besteht:
2-Hydroxy-4-[2-hydroxy-2-(5,6,7,8-tetrahydro-5,5,8,8-tetramethyl-2-naphthyl)-ethoxy]-benzoesäure,
2-Hydroxy-4-[2-hydroxy-2-(5,6,7,8-tetrahydro-5,5,8,8-tetramethyl-2-naphthyl)-ethoxy]-methylbenzoat,
2-Hydroxy-4-[2-hydroxyimino-2-(5,6,7,8-tetrahydro-5,5,8,8-tetramethyl-2-naphthyl)-ethoxy]-benzoesäure,
2-Acetyloxy-4-[2-acetyloxy-2-(5,6,7,8-tetrahydro-5,5,8,8-tetramethyl-2-naphthyl)-ethoxy]-benzoesäure,
2-Hydroxy-4-[2-acetyloxy-2-(5,6,7,8-tetrahydro-5,5,8,8-tetramethyl-2-naphthyl)-ethoxy]-benzoesäure,
2-Acetyloxy-4-[2-hydroxy-2-(5,6,7,8-tetrahydro-5,5,8,8-tetramethyl-2-naphthyl)-ethoxy]benzoesäure,
2-Hydroxy-4-[2-(5,6,7,8-tetrahydro-5,5,8,8-tetramethyl-2-naphthyl)-ethoxy]-benzoesäure,
2-Hydroxy-4-[2-hydroxy-2-(5,6,7,8-tetrahydro-5,5,8,8-tetramethyl-2-naphthyl)-ethoxy]-benzylalkohol,
2-Acetyloxy-4-[2-hydroxy-2-(5,6,7,8-tetrahydro-5,5,8,8-tetramethyl-2-naphthyl)-ethoxy]-benzylalkoholacetat,
2-Hydroxy-4-[2-hydroxy-2-(5,6,7,8-tetrahydro-5,5,8,8-tetramethyl-2-naphthyl)-ethoxy]-benzoesäurepiperidinylamid,
2-Hydroxy-4-[2-hydroxy-2-(5,6,7,8-tetrahydro-5,5,8,8-tetramethyl-2-naphthyl)ethoxy]-benzoesäuremorpholinylamid,
2-Hydroxy-4-[2-hydroxy-2-(5,6,7,8-tetrahydro-5,5,8,8-tetramethyl-2-naphthyl)-ethoxy]-benzamid,
2-Hydroxy-4-[2-hydroxy-2-(5,6,7,8-tetrahydro-5,5,8,8-tetramethyl-2-naphthyl)ethoxy]-benzoesäure-N-ethylamid,
2-Hydroxy-4-[2-hydroxy-2-(4,4,-dimethylthiochroman-6-yl)-ethoxy]-methylbenzoat,
2-Hydroxy-4-[2-hydroxy-2-(4,4,-dimethylthiochroman-6-yl)-ethoxy]-benzoesäure,
2-Hydroxy-4-[3-hydroxy-3-(5,6,7,8-tetrahydro-5,5,8,8-tetramethyl-2-naphthyl)-propyl]-benzoesäure,
2-Hydroxy-4-[2-hydroxy-2-(3,5-di-tert-butyl-4-hydroxyphenyl)-ethoxy]-benzoesäure,
2-Hydroxy-4-[2-hydroxy-2-(5,6,7,8-tetrahydro-5,5,8,8-tetramethyl-2-naphthyl)-ethoxy]-toluol,
2,6-Dihydroxy-4-[2-hydroxy-2-(5,6,7,8-tetrahydro-5,5,8,8-tetramethyl-2-naphthyl)ethyl]-methylbenzoat,
2-Hydroxy-4-[2-hydroxy-2-(3-tert-butyl-4-methoxyphenyl)-ethoxy]-benzoesäure,
2-Hydroxy-4-[2-hydroxy-2-(3-tert-butyl-4-hydroxyphenyl)-ethoxy]-benzoesäure,
(-)-Isomer der 2-Hydroxy-4-[2-hydroxy-2-(5,6,7,8-tetrahydro-5,5,8,8-tetramethyl-2-naphthyl)-ethoxy]-benzoesäure,
(+)-Isomer der 2-Hydroxy-4-[2-hydroxy-2-(5,6,7,8-tetrahydro-5,5,8,8-tetramethyl-2-naphthyl)-ethoxy]benzoesäure,
2-Hydroxy-4-[2-hydroxy-2-(3-methoxy-5,6,7,8-tetrahydro-5,5,8,8-tetramethyl-2-naphthyl)-ethoxy]-benzoesäure,
2-Methoxy-4-[2-hydroxy-2-(5,6,7,8-tetrahydro-5,5,8,8-tetramethyl-2-naphthyl)-ethyl]-benzoesäure,
2-Hydroxy-4-[2-hydroxy-2-(3-hydroxy-5,6,7,8-tetrahydro-5,5,8,8-tetramethyl-2-naphthyl)-ethoxy]-benzoesäure,
2-Hydroxy-4-[2-amino-2-(5,6,7,8-tetrahydro-5,5,8,8-tetramethyl-2-naphthyl)-ethoxy]-benzoesäure,
2-Hydroxy-4-[2-hydroxy-2-(5,6,7,8-tetrahydro-5,5,8,8-tetramethyl-2-naphthyl)-propyloxy]-benzoesäure,
2-Hydroxy-4-[2-hydroxy-2-(5,6,7,8-tetrahydro-5,5,8,8-tetramethyl-2-naphthyl)-hexyloxy]-benzoesäure,
2-Hydroxy-4-[2-hydroxy-2-(5,6,7,8-tetrahydro-5,5,8,8-tetramethyl-2-naphthyl)-ethylamino]-benzoesäure,
2-Hydroxy-4-{[2-hydroxy-2-[3-(1-adamantyl)-4-methoxyphenyl]-ethoxy]}-benzoesäure,
2-Hydroxy-4-{[2-[3-(1-adamantyl)-4-methoxyphenyl]-ethoxy]}-benzoesäure.

17. Zubereitung, dadurch gekennzeichnet, daß sie in einem geeigneten Träger für die enterale, parenterale, topische oder ophthalmologische Verabreichung mindestens eine Verbindung gemäß Formel (I) nach einem der Ansprüche 1 bis 16 enthält.

18. Zubereitung gemäß Anspruch 17, dadurch gekennzeichnet, daß sie 0,001 bis etwa 5 Gew.-% einer Verbindung gemäß Formel (I) enthält.

19. Verwendung einer Verbindung gemäß einem der Ansprüche 1 bis 16 zur Herstellung eines Arzneimittels, welches für die Behandlung dermatologischer, rheumatischer, respiratorischer und ophthalmologischer Affektionen bestimmt ist.

20. Kosmetische Zubereitung für die Körper- und Haarhygiene, dadurch gekennzeichnet, daß sie in einem kosmetisch geeigneten Träger mindestens eine Verbindung der Formel (I) gemäß einem der Ansprüche 1 bis 16 enthält.

21. Kosmetische Zubereitung gemäß Anspruch 20, dadurch gekennzeichnet, daß sie die Verbindung gemäß Formel (I) in einer Konzentration zwischen 0,001 und 3 Gew.-% enthält.

## Patentansprüche (Patentansprüche für folgende(n) Vertragsstaat(en): ES)

1. Kosmetische Zubereitung für die Körperhygiene und Haarpflege, dadurch gekennzeichnet, daß sie in einem geeigneten kosmetischen Träger mindestens eine Verbindung gemäß Formel (I) enthält: 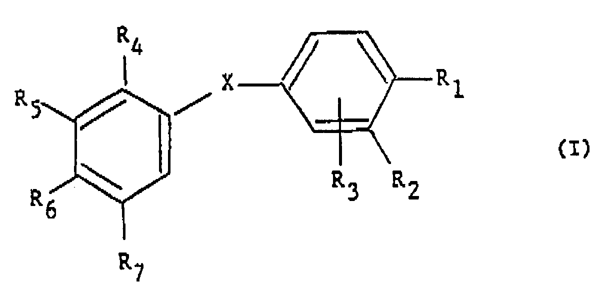 worin
R₁ die -CH₃-Gruppe, die -CH₂OH-Gruppe, den Rest -COR₈ oder den Rest -CH₂OCOR₉ bedeutet,
wobei R₈ ein Wasserstoffatom, OH, -OR₁₀, 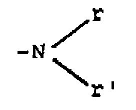 oder einen niedrigen Alkylrest bedeutet,
R₁₀ einen Alkylrest mit 1 bis 20 Kohlenstoffatomen, einen Alkenylrest mit 2 bis 20 Kohlenstoffatomen, einen Aryl- oder Aralkylrest darstellt,
r und r', die gleich oder verschieden sein können, ein Wasserstoffatom, einen niedrigen Alkylrest, einen Arylrest, einen d'α-Aminosäurerest, einen Zuckerrest oder einen Heterocyclus bedeuten, oder r und r' zusammengenommen einen Heterocyclus bilden,
R₉ einen Alkylrest mit 1 bis 20 Kohlenstoffatomen, einen Alkenylrest mit 2 bis 20 Kohlenstoffatomen oder einen Zuckerrest darstellt,
R₂ und R₃ -OR₁₁ oder -OCOR₁₁ bedeuten,
wobei R₁₁ ein Wasserstoffatom, einen niedrigen Alkylrest, einen Fluoralkylrest mit 1 bis 6 Kohlenstoffatomen und 3 bis 7 Fluoratomen, einen Aryl- oder Aralkylrest bedeutet,
R₃ weiterhin ein Wasserstoffatom bedeutet,
R₄ ein Wasserstoffatom, OH, einen niedrigen Alkylrest, einen Alkoxyrest mit 1 bis 6 Kohlenstoffatomen, ein Fluoratom, ein Chloratom oder die CF₃-Gruppe darstellt,
R₅ und R₇ ein Wasserstoffatom, OH, einen Alkoxyrest mit 1 bis 6 Kohlenstoffatomen, einen α-substituierten Alkylrest mit 3 bis 12 Kohlenstoffatomen oder einen α,α'-disubstitierten Alkylrest mit 4 bis 12 Kohlenstoffatomen, einen Cycloalkylrest mit 3 bis 12 Kohlenstoffatomen, einen mono- oder polycyclischen Rest mit 5 bis 12 Kohlenstoffatomen darstellen, der mit dem Phenylkern über ein tertiäres Kohlenstoffatom verknüpft ist, wobei R₅ und R₇ nicht gleichzeitig OH oder einen Alkoxyrest bedeuten können,
R₆ ein Wasserstoffatom, OH, einen niedrigen Alkylrest, einen Alkoxyrest mit 1 bis 6 Kohlenstoffatomen, einen Cycloalkylrest mit 3 bis 12 Kohlenstoffatomen, einen Monohydroxyalkylrest, einen Polyhydroxyalkylrest, ein Fluoratom, ein Chloratom, einen Alkenylrest mit 2 bis 6 Kohlenstoffatomen oder einen Alkenyloxyrest mit 2 bis 6 Kohlenstoffatomen darstellt, wobei R₄, R₅, R₆ und R₇ nicht gleichzeitig ein Wasserstoffatom bedeuten können, und
R₅ und R₆ oder R₆ und R₇ zusammengenommen mit dem benachbarten aromatischen Ring einen 5- oder 6-gliedrigen Ring bilden können, der gegebenenfalls durch Methylgruppen substituiert und/oder gegebenenfalls durch ein Sauerstoff- oder Schwefelatom unterbrochen sein kann,
X ein zweiwertiges Radial darstellt, welches von links nach rechts gelesen oder umgekehrt aus der Gruppe ausgewählt ist, die aus den folgenden Resten besteht:
(i) -C(R₁₃R₁₄)-C(R₁₆R₁₈)-W-
(ii) -C(R₁₄R₁₆)-W-C(R₁₈R₁₉)-
(iii) -C(R₁₃R₁₄)-C(R₁₅R₁₆)-C(R₁₈R₂₀)-
(iv) -CR₁₇=CR₂₁-C(R₁₃R₁₄)-
worin
W ein Sauerstoffatom, die -NR₁₂-Gruppe oder die S(O)n-Gruppe bedeutet, wobei n entweder 0, 1 oder 2 bedeutet,
R₁₃, R₁₅ und R₂₀ ein Wasserstoffatom, den Rest -OR₁₁, OCOR₁₁, -NHCOR₁₁, einen Rest 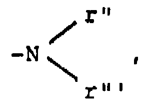 einen Aralkylrest, einen niedrigen Alkylrest, einen Monohydroxyalkylrest oder einen Polyhydroxyalkylrest bedeuten,
wobei r'' und r''', welche gleich oder verschieden sind, ein Wasserstoffatom, einen niedrigen Alkylrest, einen Alkenylrest mit 2 bis 6 Kohlenstoffatomen oder einen Alkinylrest mit 2 bis 6 Kohlenstoffatomen darstellen,
R₁₄, R₁₆, R₁₈ und R₁₉ ein Wasserstoffatom, einen Aralkylrest, einen niedrigen Alkylrest, einen Monohydroxyalkyl- oder Polyhydroxyalkylrest darstellen, wobei
unter der Maßgabe, daß X (i) bedeutet, R₁₃ und R₁₄ einen Rest =N-OR₁₁ oder einen Rest =N-OCOR₁₁ bilden können, und
unter der Maßgabe, daß X (iii) oder (iv) bedeutet, R₁₄, R₁₆ und R₁₈ jeweils den Rest -OR₁₁ oder den Rest OCOR₁₁ bedeuten können, oder R₁₃, R₁₄ oder R₁₅, R₁₆ zusammengenommen einen Rest =NOR₁₁ oder einen Rest =N-OCOR₁₁ bilden können,
wobei R₁₂ ein Wasserstoffatom, einen niedrigen Alkylrest, einen Aralkylrest, einen Alkenylrest mit 2 bis 6 Kohlenstoffatomen, einen Alkinylrest mit 2 bis 6 Kohlenstoffatomen oder einen Fluoralkylrest mit 1 bis 6 Kohlenstoffatomen und 3 bis 7 Fluoratomen darstellt,
R₁₇ ein Wasserstoffatom, eine Hydroxylgruppe, einen niedrigen Alkylrest oder einen Alkoxyrest mit 1 bis 6 Kohlenstoffatomen bedeutet,
R₂₁ ein Wasserstoffatom oder einen niedrigen Alkylrest bedeutet,
sowie die Salze der Verbindungen gemäß Formel (I) mit der Maßgabe, daß R₁ eine Carbonsäurefunktion bedeutet sowie mit der Maßgabe, daß R₁₃, R₁₅ oder R₂₀ eine Aminfunktion bedeutet sowie die optischen Isomeren von Verbindungen gemäß Formel (I).

2. Kosmetische Zubereitung nach Anspruch 1, dadurch gekennzeichnet, daß sie die Verbindung gemäß Formel (I) in einer Konzentration im Bereich 0,001 und 3 Gew.% enthält.

3. Zubereitung nach Anspruch 1, dadurch gekennzeichnet, daß die Verbindung gemäß Formel (I) in Form von Alkali- oder Erdalkalisalzen oder auch in Form von Zinksalzen oder einem organischen Amin vorliegt.

4. Zubereitung nach Anspruch 1, dadurch gekennzeichnet, daß die Verbindung gemäß Formel (I) in Form der Salze einer Mineral- oder organischen Säure vorliegt, die aus der Reihe Salzsäure, Schwefelsäure, Essigsäure, Zitronensäure, Fumarsäure, Hemibernsteinsäure, Maleinsäure und Mandelsäure ausgewählt sind.

5. Zubereitung nach einem der vorstehenden Ansprüche, dadurch gekennzeichnet, daß die Verbindung gemäß Formel (I) aus der aus den folgenden Verbindungen bestehenden Gruppe entnommen ist:
2-Hydroxy-4-[2-hydroxy-2-(5,6,7,8-tetrahydro-5,5,8,8-tetramethyl-2-naphthyl)-ethoxy]-benzoesäure,
2-Hydroxy-4-[2-hydroxy-2-(5,6,7,8-tetrahydro-5,5,8,8-tetramethyl-2-naphthyl)-ethoxy]-methylbenzoat,
2-Hydroxy-4-[2-hydroxyimino-2-(5,6,7,8-tetrahydro-5,5,8,8-tetramethyl-2-naphthyl)-ethoxy]-benzoesäure,
2-Acetyloxy-4-[2-acetyloxy-2-(5,6,7,8-tetrahydro-5,5,8,8-tetramethyl-2-naphthyl)-ethoxy]-benzoesäure,
2-Hydroxy-4-[2-acetyloxy-2-(5,6,7,8-tetrahydro-5,5,8,8-tetramethyl-2-naphthyl)-ethoxy]-benzoesäure,
2-Acetyloxy-4-[2-hydroxy-2-(5,6,7,8-tetrahydro-5,5,8,8-tetramethyl-2-naphthyl)-ethoxy]-benzoesäure,
2-Hydroxy-4-[2-(5,6,7,8-tetrahydro-5,5,8,8-tetramethyl-2-naphthyl)-ethoxy]-benzoesäure,
2-Hydroxy-4-[2-hydroxy-2-(5,6,7,8-tetrahydro-5,5,8,8-tetramethyl-2-naphthyl)-ethoxy]-benzylalkohol,
2-Acetyloxy-4-[2-hydroxy-2-(5,6,7,8-tetrahydro-5,5,8,8-tetramethyl-2-naphthyl)-ethoxy]benzylalkoholacetat,
2-Hydroxy-4-[2-hydroxy-2-(5,6,7,8-tetrahydro-5,5,8,8-tetramethyl-2-naphthyl)-ethoxy]benzoesäurepiperidinylamid,
2-Hydroxy-4-[2-hydroxy-2-(5,6,7,8-tetrahydro-5,5,8,8-tetramethyl-2-naphthyl)-ethoxy]-benzoesäuremorpholinylamid,
2-Hydroxy-4-[2-hydroxy-2-(5,6,7,8-tetrahydro-5,5,8,8-tetramethyl-2-naphthyl)-ethoxy]-benzamid,
2-Hydroxy-4-[2-hydroxy-2-(5,6,7,8-tetrahydro-5,5,8,8-tetramethyl-2-naphthyl)-ethoxy]-benzoesäure-N-ethylamid,
2-Hydroxy-4-[2-hydroxy-2-(4,4,-dimethylthiochroman-6-yl)-ethoxy]-methylbenzoat,
2-Hydroxy-4-[2-hydroxy-2-(4,4,-dimethylthiochroman-6-yl)-ethoxy]-benzoesäure,
2-Hydroxy-4-[3-hydroxy-3-(5,6,7,8-tetrahydro-5,5,8,8-tetramethyl-2-naphthyl)-propyl]-benzoesäure,
2-Hydroxy-4-[2-hydroxy-2-(3,5-di-tert-butyl-4-hydroxyphenyl)-ethoxy]-benzoesäure,
2-Hydroxy-4-[2-hydroxy-2-(5,6,7,8-tetrahydro-5,5,8,8-tetramethyl-2-naphthyl)-ethoxy]-toluol,
2,6-Dihydroxy-4-[2-hydroxy-2-(5,6,7,8-tetrahydro-5,5,8,8-tetramethyl-2-naphthyl)-ethyl]-methylbenzoat,
2-Hydroxy-4-[2-hydroxy-2-(3-tert-butyl-4-methoxyphenyl)-ethoxy]-benzoesäure,
2-Hydroxy-4-[2-hydroxy-2-(3-tert-butyl-4-hydroxyphenyl)-ethoxy]-benzoesäure;
(-)-Isomer der 2-Hydroxy-4-[2-hydroxy-2-(5,6,7,8-tetrahydro-5,5,8,8-tetramethyl-2-naphthyl)-ethoxy]-benzoesäure,
(+)-Isomer der 2-Hydroxy-4-[2-hydroxy-2-(5,6,7,8-tetrahydro-5,5,8,8-tetramethyl-2-naphthyl)-ethoxy]-benzoesäure,
2-Hydroxy-4-[2-hydroxy-2-(3-methoxy-5,6,7,8-tetrahydro-5,5,8,8-tetramethyl-2-naphthyl)-ethoxy]-benzoesäure,
2-Methoxy-4-[2-hydroxy-2-(5,6,7,8-tetrahydro-5,5,8,8-tetramethyl-2-naphthyl)-ethyl]-benzoesäure,
2-Hydroxy-4-[2-hydroxy-2-(3-hydroxy-5,6,7,8-tetrahydro-5,5,8,8-tetramethyl-2-naphthyl)-ethoxy]-benzoesäure,
2-Hydroxy-4-[2-amino-2-(5,6,7,8-tetrahydro-5,5,8,8-tetramethyl-2-naphthyl)-ethoxy]-benzoesäure,
2-Hydroxy-4-[2-hydroxy-2-(5,6,7,8-tetrahydro-5,5,8,8-tetramethyl-2-naphthyl)-propyloxy]-benzoesäure,
2-Hydroxy-4-[2-hydroxy-2-(5,6,7,8-tetrahydro-5,5,8,8-tetramethyl-2-naphthyl)-hexyloxy]-benzoesäure,
2-Hydroxy-4-[2-hydroxy-2-(5,6,7,8-tetrahydro-5,5,8,8-tetramethyl-2-naphthyl)-ethylamino]-benzoesäure,
2-Hydroxy-4-{[2-hydroxy-2-[3-(1-adamantyl)-4-methoxyphenyl]-ethoxy)}-benzoesäure,
2-Hydroxy-4-{[2-[3-(1-adamantyl)-4-methoxyphenyl]-ethoxy]}-benzoesäure.
